# EUROPEAN PATENT SPECIFICATION

(11) **EP 1 933 139 B1**
(45) Date of publication and mention of the grant of the patent: **28.09.2011**
(21) Application number: 06025522.1
(22) Date of filing: 11.12.2006
(51) Int. Cl.: G01N 33/558, G01N 33/543, G01N 33/58, C12Q 1/68

(54) **Rapid immunochromatographic detection by amplification of the colloidal gold signal**
Schnelle immunochromatographische Detektion durch Verstärkung des kolloidalen Gold Signals
Détection rapide immunochromatographique par amplification du signal d'or colloïdal

(43) Date of publication of application: 18.06.2008
(73) Proprietor: AraGen Biotechnology Co. Ltd., 11710 Naor (JO)
(72) Inventor: Badwan, Adnan, Dr., 11185 Amman (JO); Murshed, Abedel-qader Mohammed, 11118 Amman (JO)
(74) Representative: Winkler, Andreas Fritz Ernst

(56) References cited:
- WO-A-02/46472
- GB-A- 2 261 284
- TANAKA R ET AL: "A novel enhancement assay for immunochromatographic test strips using gold nanoparticles." ANALYTICAL AND BIOANALYTICAL CHEMISTRY, vol. 385, no. 8, August 2006 (2006-08), pages 1414-1420, XP002424110
- OKU Y ET AL: "Development of oligonucleotide lateral-flow immunoassay for multi-parameter detection" JOURNAL OF IMMUNOLOGICAL METHODS, vol. 258, no. 1-2, 1 December 2001 (2001-12-01), pages 73-84, XP004311918
- SIMS P W ET AL: "IMMUNOPOLYMERASE CHAIN REACTION USING REAL-TIME POLYMERASE CHAIN REACTION FOR DETECTION" ANALYTICAL BIOCHEMISTRY, vol. 281, no. 2, 1 June 2000 (2000-06-01), pages 230-232, XP001147707
- HAZARIKA P. ET AL: 'Sensitive detection of proteins using difunctional DNA-gold nanoparticles' SMALL, WILEY - VCH VERLAG GMBH & CO. KGAA, DE vol. 1, no. 8-9, 01 January 2005, pages 844 - 848, XP002407187 ISSN: 1613-6810

## Description

The present invention relates in general to the field of diagnostics, namely to a device for the detection of more than one target in a sample, preferably in urine. More precisely, the present invention relates to a high sensitive rapid immunochromatographic test device preferably providing a unique system for the detection of more than one target in a single assay at the same time. The present invention further refers to a method for the production of the test device, to the uses of the test device for the detection of more than one disease infection such as Human Immunodeficiency virus (HIV) and Tuberculosis Lipoarabinomannan (LAM) at the same time in urine, as well as to a kit comprising the test device.

### BACKGROUND OF THE INVENTION

In recent years the *in vitro* diagnostics (IVD) industry has made enormous efforts to develop immunochromatographic tests. Such tests have found applications in both clinical and non-clinical fields ¹. A clinical utility of this test format has been shown for more than 150 different analytes, and many of them are target now of commercially available diagnostic products ³. The wide range of applications for such devices has been reviewed ^{1,2}.

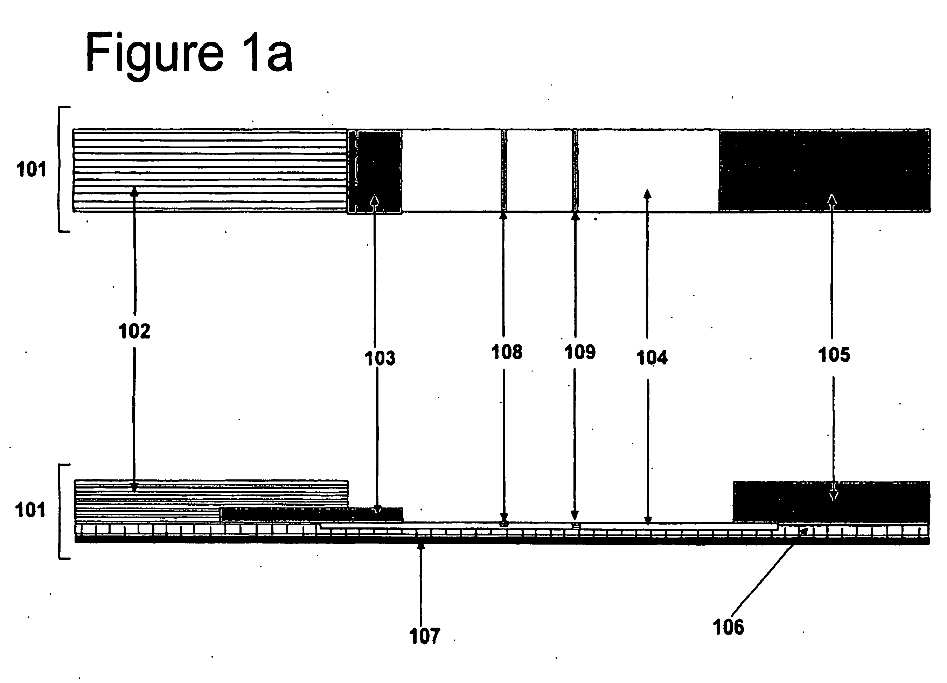

Rapid immunochromatographic test devices, e.g. in the form of a test strip, are made up of a number of components (Figure 1a). Such a test strip 101 commonly includes a sample pad 102, a conjugate pad 103, a membrane 104, e.g. a nitrocellulose membrane, and an absorbent pad 105. The membrane 104 is usually attached by means of an adhesive 106 to a supporting backing 107, e.g. made of plastic. In practice, the user dispense a patient sample (usually urine or whole blood) onto the sample pad 102. The sample then flows through the sample pad 102 into the conjugate pad 103, where it mixes with and releases the detector reagent. This mixture then flows across the membrane 104, where it binds with the test and control reagents located in the capture test zone 108 (sample zones) and negative control zone 109, respectively. When the mixture binds to the reagent that forms the test line, a positive result is indicated. The colour intensity of the test line is proportional to the concentration of analyte in the sample. Excess sample that flows beyond the test and control zones 108, 109 is taken up in the absorbent pad 105.

Rapid immunochromatographic test devices for diagnostic purposes are easy to operate and thus do not only contribute to the comfort of professional users, e.g. medical stuff, but also allow the operation by non-professionals users, e.g. most patients.

Using rapid immunochromatographic tests, a physician can instantly not only examine a sample by himself, but also diagnose a patient on the basis of the obtained results. The main disadvantage of the one rapid immunochromatographic test is that it only detects one parameter at a time. Thus, many different rapid immunochromatographic test devices are needed to check the suspected parameters, resulting in the requirement of enormous sample volume obtained from a patient as well as cumbersome test procedures.

In addition, the use of urine samples may be attractive due to the ease of sample collection, possible cost savings, better safety (against needle-stick injuries) and higher compliance rates. Assays for this type of specimens might be a useful alternative when it is for instance difficult or impossible to perform HIV-tests in blood samples. Further, it might be possible that blood cannot be drawn for religious reasons or that difficulties may be experienced in collecting blood samples. Therefore, urinary samples are the more safety type of specimens in most of infection cases.

UK 2 261 284 refers to a device for performing biochemical diagnostic assays which comprises two liquid flow channels of porous material which transfer liquid by capillary flow to a common site in sequentially timed manner following simultaneous application of the liquid to the end of the channels. A number of channel pairs can be formed in the layers for simultaneously performing plural test procedures. Typical use is an immunoassay using serum or urine and immobilized antibody binding.

WO 02/46472 refers to the detection of analytes, in particular nucleic acids. The method comprises contacting the nucleic acid with particles having oligonucleotides attached thereto.

Tanaka *et al.* 2006 ²¹ refers to a highly sensitive immunochromatographic assay applied to detect hCG as the model case. Primary antibody-conjugated gold nanoparticles were used as an enhancer. The primary antibodies were immobilized within a defined detection zone (test line) on the diagnostic nitrocellulose membrane. Secondary antibodies were conjugated with colloidal gold nanoparticles. The gold-conjugated antibodies and the primary antibodies formed a sandwich complex with the target protein. Within the test line, the sandwich complex was immobilized.

Hazarika *et al.* 2005 ²² refers to the sensitive detection of proteins using an alternative approach based on difunctional DNA-gold nanoparticles.

Thus, there is a need to develop a multi-parameter rapid immunochromatographic detection test device suitable to detect more than two types of antigens and/or antibodies in a single assay at the same time. Further there is a need in the prior art to develop a multi-parameter rapid immunochromatographic detection test device sensitive enough to detect more than two types of antigens and/or antibodies in a sample such as urine.

Thus, it is an object of the present invention to provide a rapid immunochromatographic detection test overcoming the drawbacks of the prior art, being preferably suitable for ultra sensitive detection of at least one antibody and at least one antigen, preferably in urine at the same time.

### SUMMARY OF THE INVENTION

In one embodiment the present invention concerns a rapid immunochromatographic test device for the detection of more than one target in a sample, comprising
(a) a first gold conjugate releasing pad, comprising at least two colloidal gold conjugates I and II, wherein colloidal gold conjugate I is conjugated with a first antibody capturing a target antigen from a first site and at least one oligonucleotide, and wherein colloidal gold conjugate II is conjugated with a first antigen being captured by a target antibody or second antibody being specific for said target antibody and at least one oligonucleotide, wherein the oligonucleotide of colloidal gold conjugate II differs from the oligonucleotide of colloidal gold conjugate I;
   wherein the colloidal gold conjugates I and II form "target-gold conjugate I" and "target-gold conjugate II";
(b) a second gold conjugate releasing pad, comprising at least two colloidal gold conjugates III and IV, wherein colloidal gold conjugate III is conjugated with a third antibody capturing the target antigen from a second site and at least one oligonucleotide, wherein the oligonucleotide of colloidal gold conjugate III is complementary to the oligonucleotide of colloidal gold conjugate I, and wherein colloidal gold conjugate IV is conjugated with a second antigen being captured by the same target antibody that captures the first antigen or fourth antibody being specific for said target antibody and at least one oligonucleotide, wherein the oligonucleotide of colloidal gold conjugate IV is complementary to the oligonucleotide of colloidal gold conjugate II;
   wherein both gold conjugate releasing pads are located at different positions within the test device;
(c) a test strip comprising a sample pad, a conjugate pad comprising said first gold conjugate releasing pad, a conjugate pad comprising said second gold conjugate releasing pad, a membrane comprising two capture test zones and a negative control zone, and an absorbent pad;
   wherein one capture test zone comprises said third antibody and wherein the other capture test zone comprises said second antigen or fourth antibody;
such that after the "target-gold conjugate I" and "target-gold conjugate II" are captured within the two capture test zones, the second gold conjugate releasing pad will release the colloidal gold conjugates III and IV.

In another embodiment the present invention relates to a rapid immunochromatographic test device for the detection of more than one target in a sample, comprising
(a) a first gold conjugate releasing pad, comprising at least two colloidal gold conjugates I and II, wherein colloidal gold conjugate I is conjugated with a first antibody capturing a target antigen from a first site and at least one further antibody or antigen (A-1), and wherein colloidal gold conjugate II is conjugated with a first antigen being captured by a target antibody or second antibody being specific for said-target antibody and at least one further antibody or antigen (A-2), wherein said antibodies or antigens (A-1) and (A-2) differ from each other and differ from the first and second antigens and antibodies;
   wherein the colloidal gold conjugates I and II form "target-gold conjugate I" and "target-gold conjugate II";
(b) a second gold conjugate releasing pad comprising at least two colloidal gold conjugates III and IV, wherein colloidal gold conjugate III is conjugated with a third antibody capturing the target antigen from a second site and at least one further antibody or antigen (A-3), wherein said antibody or antigen (A-3) is complementary to said antibody or antigen (A-1), and wherein colloidal gold conjugate IV is conjugated with a second antigen being captured by the same target antibody that captures the first antigen or fourth antibody being specific for said target antibody and at least one further antibody or antigen (A-4), wherein said antibody or antigen (A-4) is complementary to said antibody or antigen (A-2);
   wherein both gold conjugate releasing pads are located at different positions within the test device.
(c) a test strip comprising a sample pad, a conjugate pad comprising said first gold conjugate releasing pad, a conjugate pad comprising said second gold conjugate releasing pad, a membrane comprising two capture test zones and a negative control zone, and an absorbent pad;
   wherein one capture test zone comprises said third antibody and wherein the other capture test zone comprises said second antigen or fourth antibody;
such that after the "target-gold conjugate I" and "target-gold conjugate II" are captured within the two capture test zones, the second gold conjugate releasing pad will release the colloidal gold conjugates III and IV.

In a further embodiment the present invention concerns a method for the production of a device according to the present invention comprising the steps of
a) preparing a colloidal gold solution;
b) preparing a conjugation buffer;
c) partitioning the conjugation buffer by dividing it into a first, second, third and fourth flask;
d) adding an antibody according to claim 8 to the conjugation buffer in the first flask;
e) adding an antigen according to claim 9 to the conjugation buffer in the second flask;
f) adding an antibody according to claim 8 to the conjugation buffer in the third flask, wherein said antibody differs from the antibody used in step d);
g) adding an antigen according to claim 9 to the conjugation buffer in the fourth flask, wherein said antigen differs from the antigen used in step e);
h) preparing and adding oligonucleotides labelled BSA aqueous solution to the first flask;
i) preparing and adding oligonucleotides labelled BSA aqueous solution to the second flask, wherein said oligonucleotides differ from the oligonucleotides used in step h);
j) preparing and adding oligonucleotides labelled BSA aqueous solution to the third flask, wherein said oligonucleotides are complementary to the oligonucleotides used in step h);
k) preparing and adding oligonucleotides labelled BSA aqueous solution to the fourth flask, wherein said oligonucleotides are complementary to the oligonucleotides used in step i);
l) adding colloidal gold solution into each flask;
m) adding stabilizing buffer to each flask;
n) concentrating each conjugate;
o) mixing conjugates of the first and second flask; adding a surfactant and soaking a glass fibre sheet conjugate pad into the conjugate;
p) mixing conjugates of the third and fourth flask; soaking another glass fibre sheet conjugate pad into the conjugate;
q) printing capturing/sample and control lines onto the membrane,
r) laminating cards; and
s) cutting cards into strips.
or comprising the steps of
a) preparing a colloidal gold solution;
b) preparing a conjugation buffer;
c) partitioning the conjugation buffer by dividing it into a first, second, third and fourth flask;
d) adding an antibody according to claim 8 to the conjugation buffer in the first flask;
e) adding an antigen according to claim 9 to the conjugation buffer in the second flask;
f) adding an antibody according to claim 8 to the conjugation buffer in the third flask, wherein said antibody differs from the antibody used in step d);
g) adding an antigen according to claim 9 to the conjugation buffer in the fourth flask, wherein said antigen differs from the antigen used in step e);
h) preparing and adding aqueous solution comprising antibodies or antigens to the first flask, wherein said antibodies or antigens are different from the antibodies or antigens used in step d), e), g) and f);
i) preparing and adding aqueous solution comprising antibodies or antigens to the second flask, wherein said antibodies or antigens are different from the antibodies or antigens used in step d), e), g) and f) and of the antibodies used in step h);
j) preparing and adding aqueous solution comprising antibodies or antigens to the third flask, wherein said antibodies or antigens are complementary to the antibodies or antigens used in step h);
k) preparing and adding aqueous solution comprising antibodies or antigens to the fourth flask, wherein said antibodies or antigens are complementary to the antibodies or antigens used in step i);
l) adding colloidal gold solution into each flask;
m) adding stabilizing buffer to each flask;
n) concentrating each conjugate;
o) mixing conjugates of the first and second flask; adding a surfactant and soaking a glass fibre sheet conjugate pad into the conjugate;
p) mixing conjugates of the third and fourth flask; soaking another glass fibre sheet conjugate pad into the conjugate;
q) printing capturing/sample and control lines onto the membrane;
r) laminating cards; and
s) cutting cards into strips.

In another embodiment the present invention relates to the use of a device according to the present invention for the detection of at least two diseases in at least one sample.

In a further embodiment the present invention refers to a kit for detection of a disease comprising the device according to the present invention and a manual

### DESCRIPTION OF THE PREFERRED EMBODIMENTS OF THE INVENTION

Before the present invention is described in more detail below, it is to be understood that this invention is not limited to the particular methodology, protocols and reagents described herein as these may vary. It is also to be understood that the terminology used herein is for the purpose of describing particular embodiments only, and is not intended to limit the scope of the present invention which will be limited only by the appended claims. Unless defined otherwise, all technical and scientific terms used herein have the same meanings as commonly understood by one of ordinary skill in the art.

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step.

As outlined above there is a need in the prior art to provide a new test device suitable for the detection of at least one antibody and one, preferably in urine at the same time.

In a first aspect the present invention provides a rapid immunochromatographic test device for the detection of more than one target in a sample, comprising
(a) a first gold conjugate releasing pad, comprising at least two colloidal gold conjugates I and II, wherein colloidal gold conjugate I is conjugated with a first antibody capturing a target antigen from a first site and at least one oligonucleotide, and wherein colloidal gold conjugate II is conjugated with a first antigen being captured by a target antibody or second antibody being specific for said target antibody and at least one oligonucleotide, wherein the oligonucleotide of colloidal gold conjugate II differs from the oligonucleotide of colloidal gold conjugate I;
   wherein the colloidal gold conjugates I and II form "target-gold conjugate I" and "target-gold conjugate II";
(b) a second gold conjugate releasing pad, comprising at least two colloidal gold conjugates III and IV, wherein colloidal gold conjugate III is conjugated with a third antibody capturing the target antigen from a second site and at least one oligonucleotide, wherein the oligonucleotide of colloidal gold conjugate III is complementary to the oligonucleotide of colloidal gold conjugate I, and wherein colloidal gold conjugate IV is conjugated with a second antigen being captured by the same target antibody that captures the first antigen or fourth antibody being specific for said target antibody and at least one oligonucleotide, wherein the oligonucleotide of colloidal gold conjugate IV is complementary to the oligonucleotide of colloidal gold conjugate II;
   wherein both gold conjugate releasing pads are located at different positions within the test device;
(c) a test strip comprising a sample pad, a conjugate pad comprising said first gold conjugate releasing pad, a conjugate pad comprising said second gold conjugate releasing pad, a membrane comprising two capture test zones and a negative control zone, and an absorbent pad;
   wherein one capture test zone comprises said third antibody and wherein the other capture test zone comprises said second antigen or fourth antibody;
such that after the "target-gold conjugate I" and "target-gold conjugate II" are captured within the two capture test zones, the second gold conjugate releasing pad will release the colloidal gold conjugates III and IV.

The first and second colloidal (gold I and II) will capture the antigen/antibody in the sample and form the complexes "target-gold conjugate I" and "target-gold conjugate II". Preferably the targets in the sample, preferably urine, are an antigen and/or antibody.

In one preferred embodiment of the device according to the present invention the first antibody of gold I will capture the target antigen from a first site (let us consider this site as site A), the first antigen of gold II will be captured by the target antibody, the third antibody of gold III will capture the target antigen from a second site (let us consider this site as site B), and the second antigen of gold IV will be captured by the same target antibody that capture the first antigen as mentioned above.

In another preferred embodiment of the device according to the present invention each gold conjugate comprises between 1 and 6 different oligonucleotides. Preferably each gold conjugate comprises between 2 and 4 different oligonucleotides. These oligonucleotides usually have a length of about 15 to 25 nucleotides, preferably of about 20 nucleotides. Further these oligonucleotides have an amino group at the 5' terminus, preferably conjugated with bovine serum albumin. The bonds formed between the gold and oligonucleotides are the same bonds as those usually formed between gold and antibodies/antigens, and which are known to a person skilled in the art. Preferably such bonds are driven by hydrophobic, hydrophilic and dative binding forces.

In one preferred embodiment of the device according to the present invention the first gold conjugate releasing pad contains the gold conjugates I and II. Gold conjugate I is specific for an antigen, preferably the antigen is a target in a sample. Therefore gold I is conjugated with "clone one". Please note, that the numbering of clones are only for explanation and to recognize that always two different clones of monoclonal antibodies were used. Preferably, the two monoclonal antibodies capture the target antigen from two different sites, so they were called as a pair of monoclonal antibodies. In addition, "clone one" is specific for one site (A) of the antigen. At the same time gold I is conjugated with some oligonucleotides (0-1). Gold conjugate II is specific for an antibody, preferably the antibody is a target in a sample, and is conjugated with an antigen or a specific antibody for the antigen-specific antibody and is at the same time conjugated with some oligonucleotides (O-2) which are different from the oligonucleotides (O-1).

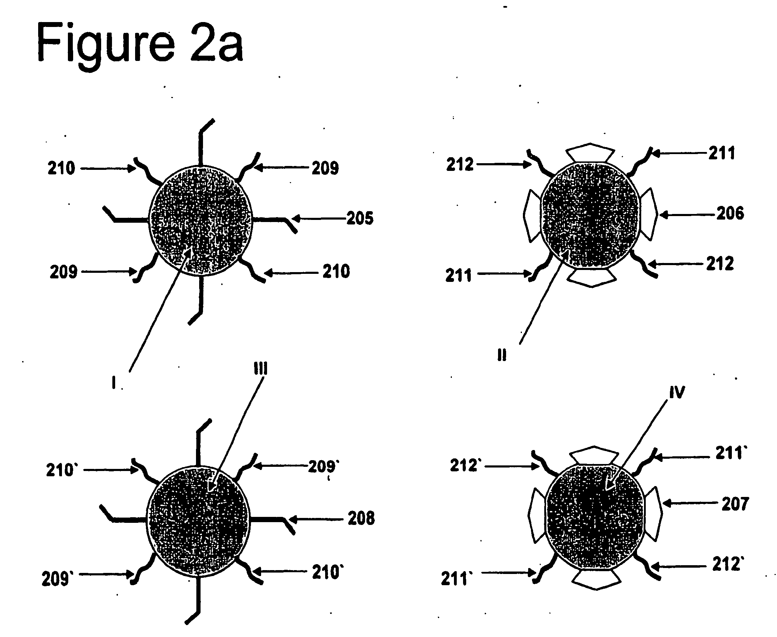

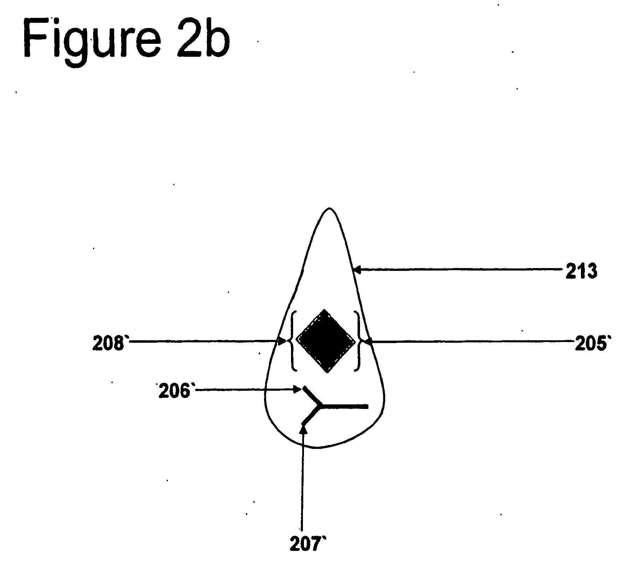

In a further preferred embodiment the second conjugate releasing pad contains the two gold conjugates III and IV. Gold conjugate III is specific for an antigen, preferably the antigen is a target in a sample, and therefore is conjugated with "clone two", whereas "clone two" is specific for the second site (B) of the antigen. At the same time gold III is conjugated with the complementary oligonucleotides (O-3) of that in the related conjugate I in the first conjugate pad. Gold conjugate IV is specific for an antibody, preferably the antibody is a target in a sample, and conjugated with the antigen or specific antibody for the antigen-specific antibody and at the same time conjugated with the complementary oligonucleotides (O-3) of that in the related conjugate II in the first conjugate pad (figure 2a and figure 2b). All used oligonucleotides should be tested for non-specific interaction theoretically and experimentally.

In one preferred embodiment of the device according to the present invention the rapid immunochromatographic test device for the detection of more than one target in a sample, comprises
c) a first gold conjugate releasing pad, comprising at least two colloidal gold conjugates I and II, wherein gold I is conjugated with a first antibody 205 and two different oligonucleotides 209, 210, and wherein gold II is conjugated with a first antigen 206 and two different oligonucleotides 211, 212, wherein the oligonucleotides 211, 212 of gold II differs from the oligonucleotides 209, 210 of gold I, and
d) a second gold conjugate releasing pad, comprising at least two colloidal gold conjugates III and IV, wherein gold III is conjugated with a third antibody 208 and two different oligonucleotides 209', 210', wherein the oligonucleotides 209', 210' of gold III are complementary to the oligonucleotides 209, 210 of gold I, and wherein gold IV is conjugated with a second antigen 207 and two oligonucleotides 211', 212', wherein the oligonucleotides 211', 212' of gold IV are complementary to the oligonucleotides 211, 212 of gold II;
   wherein both releasing pads are located at different positions within the test device (figure 2a).

In one preferred embodiment of the device according to the present invention the first antibody 205 of gold I will capture the target antigen from a first site 205', the a first antigen 206 of gold II will be captured by the target antibody 206', the third antibody 208 of gold III will capture the target antigen from a second site 208', and the second antigen 207 of gold IV will be captured by the same target antibody 207' that capture the first antigen as mentioned above, (figures 2a and 2b).

In a second aspect the present invention provides a rapid immunochromatographic test device for the detection of more than one target in a sample, comprising
(a) a first gold conjugate releasing pad, comprising at least two colloidal gold conjugates I and II, wherein colloidal gold conjugate I is conjugated with a first antibody capturing a target antigen from a first site and at least one further antibody or antigen (A-1), and wherein colloidal gold conjugate II is conjugated with a first antigen being captured by a target antibody or second antibody being specific for said-target antibody and at least one further antibody or antigen (A-2), wherein said antibodies or antigens (A-1) and (A-2) differ from each other and differ from the first and second antigens and antibodies;
   wherein the colloidal gold conjugates I and II form "target-gold conjugate I" and "target-gold conjugate II";
(b) a second gold conjugate releasing pad comprising at least two colloidal gold conjugates III and IV, wherein colloidal gold conjugate III is conjugated with a third antibody capturing the target antigen from a second site and at least one further antibody or antigen (A-3), wherein said antibody or antigen (A-3) is complementary to said antibody or antigen (A-1), and wherein colloidal gold conjugate IV is conjugated with a second antigen being captured by the same target antibody that captures the first antigen or fourth antibody being specific for said target antibody and at least one further antibody or antigen (A-4), wherein said antibody or antigen (A-4) is complementary to said antibody or antigen (A-2);
   wherein both gold conjugate releasing pads are located at different positions within the test device.
(c) a test strip comprising a sample pad, a conjugate pad comprising said first gold conjugate releasing pad, a conjugate pad comprising said second gold conjugate releasing pad, a membrane comprising two capture test zones and a negative control zone, and an absorbent pad;
   wherein one capture test zone comprises said third antibody and wherein the other capture test zone comprises said second antigen or fourth antibody;
such that after the "target-gold conjugate I" and "target-gold conjugate II" are captured within the two capture test zones, the second gold conjugate releasing pad will release the colloidal gold conjugates III and IV.

Preferably, the antibodies or antigens (A-1 to A-4) differ from said first antibody or antigen. The first antibody or antigen captures the target from the sample, whereas the other antibodies (A-1 to A-4) only function to amplify the signal by recognizing the specifically-related antibodies or antigens (A-1 to A-4). In addition, the antibodies or antigens (A-1 and A-2) conjugated with gold conjugates I and II are specifically-related to the antibodies or antigens (A-3 and A-4) of the gold conjugates III and IV, respectively.

In one preferred embodiment of the device according to the present invention the device is suitable to detect the targets in urine.

The device according to the present invention comprises a test strip comprising
a) a sample pad,
b) a conjugate pad comprising the first gold conjugate releasing pad,
c) a conjugate pad comprising the second gold conjugate releasing pad,
d) a membrane comprising two capture test zones and a negative control zone, and
e) an absorbent pad.

One capture test zone of the device according to the present invention suitable to detect an antigen in a sample comprises a third antibody specific for one site of the antigen, and the other capture test zone suitable to detect an antibody in a sample comprises a second antigen or a fourth antibody.

In another preferred embodiment the antibodies or antigens within the test zones are immobilized.

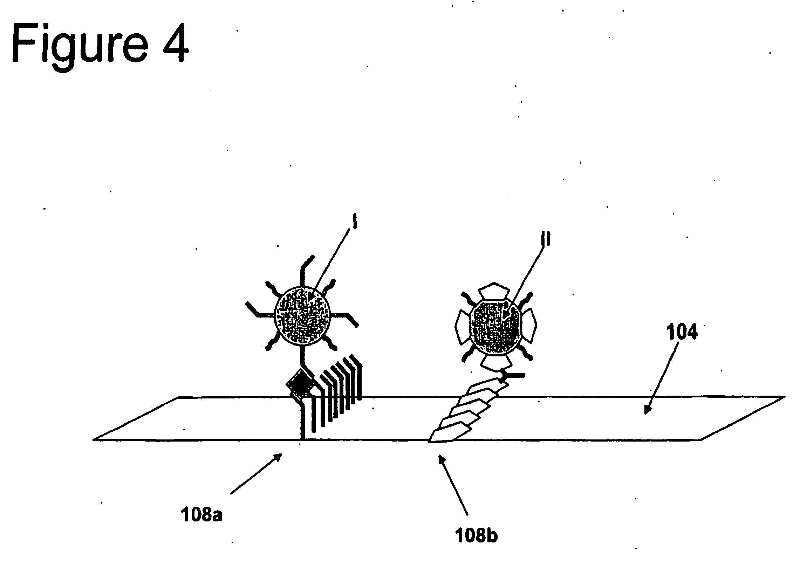

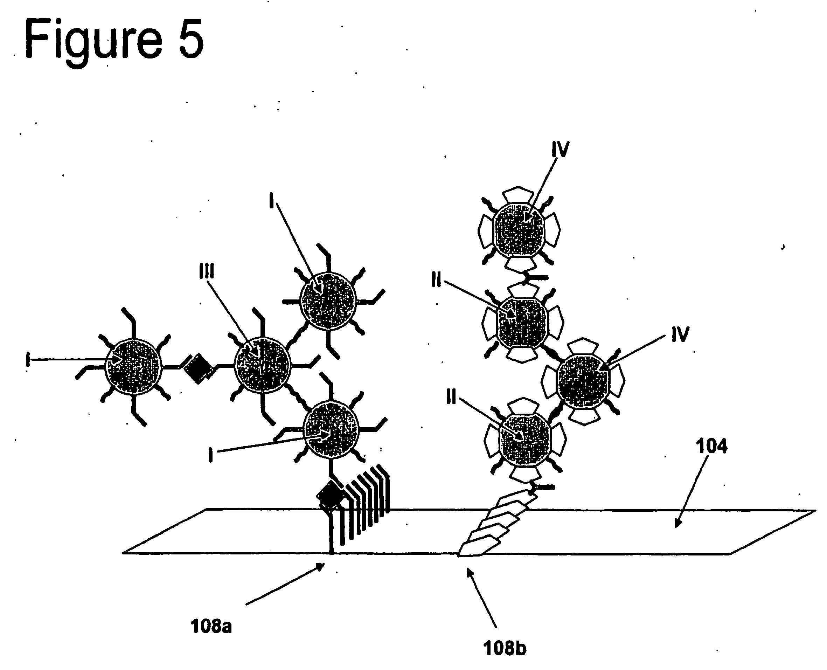

The complexes "target-gold conjugate I" and "target-gold conjugate II will be captured by the specific antibodies or antigens within the test zones and therefore be kept within these test zones to form the sandwich detection (figure 4). Then, the second gold conjugate releasing pad will release its gold conjugated with the other specific antibodies or antigens to capture the target analyte from the second site (let us consider it as site B) and at the same time with the complementary oligonucleotides that are conjugated with the colloidal gold conjugate. The last mentioned conjugate would bind with the first conjugate from different sites, this binding could be happened between any of the conjugated oligonucleotides with its complementary oligonucleotide on the other gold conjugate or between any free site B of the analyte with its specific antibody or antigen on the conjugate (figure 5). Nevertheless, the other oligonucleotides will be able to link with their complementary oligonucleotides beside the probability of capturing the first conjugate that will capture the second conjugate to form more and more branched bonds that propagate the accumulation of colloidal gold particles onto the capturing/sample line (figure 5). This propagation and accumulation of colloidal gold signal will amplify the signal and highly increase the sensitivity. This will enable us to detect very low concentrations that are not detectable using the same technique without signal amplification.

In one embodiment of the device according to the present invention the membrane is attached by means of an adhesive to a supporting backing. Preferably an acrylic pressure sensitive adhesive as known in the art is used.

In another embodiment of the device according to the present invention the first and second gold conjugate pad are laminated between the sample pad and the membrane, wherein the two gold conjugates are separated by a divider.

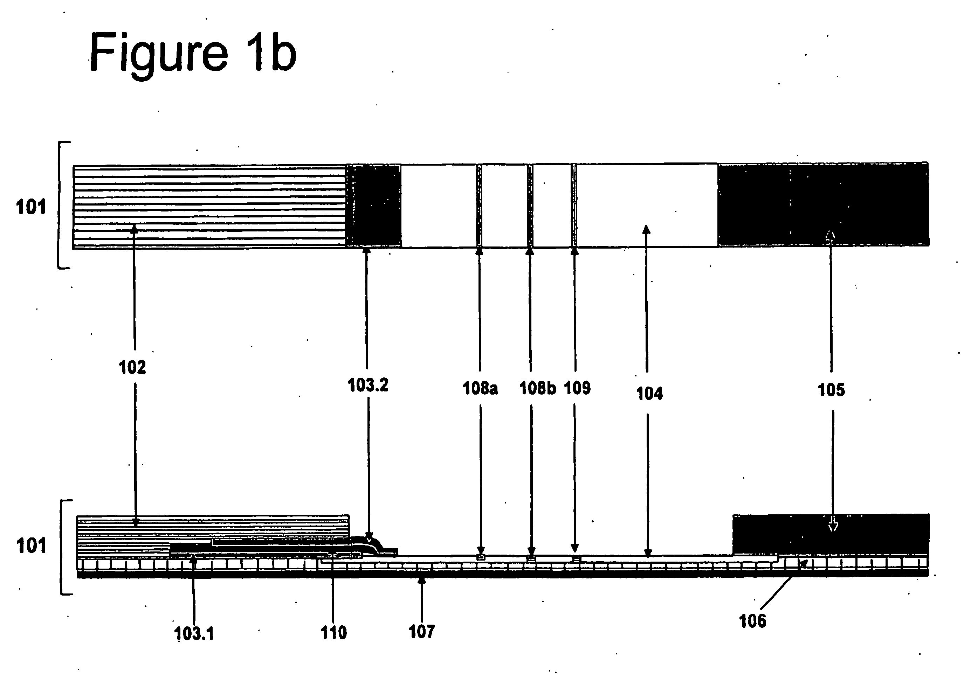

In a preferred embodiment of the device according to the present invention the first 103.1 and second gold conjugate pad 103.2 are laminated between the sample pad 102 and the membrane 104, wherein the two gold conjugates are separated by a divider 110 (Figure 1b). Preferably, the divider is an inert divider, more preferably the divider is a plastic divider

In another embodiment, the device according to the present invention the first gold conjugate pad is attached between the sample pad and the membrane while the second gold conjugate pad is within the upper part of the plastic housing to be released after sample application onto the nitrocellulose membrane directly.

In one preferred embodiment of the device according to the present invention the supporting backing is a plastic backing.

In another preferred embodiment of the device according to the present invention the membrane is nitrocellulose membrane.

In one embodiment of the device according to the present invention the first or second antibody is selected from the group comprising mouse anti-HIV p24, mouse anti-HBsAg, anti-hlgG, anti-Lipoarabinomannan, anti-H. pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

In a preferred embodiment of the device according to the present the first or second antibody is a monoclonal or polyclonal antibody, preferably a monoclonal antibody.

In another embodiment of the device according to the present invention the first antigen is selected from the group comprising conjugate of HIV antigen, conjugate of hepatitis C antigen, HIV 1 antigen, HIV 2 antigen, Lipoarabinomannan, H. pylori antigen, Toxoplasma antigen.

In a further embodiment of the device according to the present invention the control zone comprises a non-specific capturing antibody and/or a non-specific antibody capturing protein.

In one preferred embodiment of the device according to the present invention the non-specific antibody is selected from the group consisting of anti-mouse IgG, anti-rabbit IgG, anti-goat IgG, anti-donkey IgG, Anti-sheep IgG, anti-HIV p24, anti-Lipoarabinomannan, anti-H. pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

In another preferred embodiment, of the device according to the present invention the non-specific capturing protein is either Protein A or Protein G.

In a preferred embodiment of the device according to the present invention the device comprises a housing comprising at least one test strip according to the present invention.

In another preferred embodiment of the device according to the present invention the housing comprises two, three, four, five, six, seven, eight, nine, or ten test strips. Preferably the housing comprises two, three, four, or five test strips, more preferably the housing comprises two or three test strips.

In one preferred embodiment of the device according to the present invention each test strip comprises at least four antibodies or antigens, wherein two of these antibodies or antigens are immobilized onto the membrane and at the same time are conjugated with the two colloidal gold conjugates and wherein the other two antibodies or antigens are conjugated with the other two gold conjugates.

In another aspect the present invention concerns a method for the production of a device according to the present invention, comprising the steps of
a) preparing a colloidal gold solution;
b) preparing a conjugation buffer;
c) partitioning the conjugation buffer by dividing it into a first, second, third and fourth flask;
d) adding an antibody according to claim 8 to the conjugation buffer in the first flask;
e) adding an antigen according to claim 9 to the conjugation buffer in the second flask;
f) adding an antibody according to claim 8 to the conjugation buffer in the third flask, wherein said antibody differs from the antibody used in step d);
g) adding an antigen according to claim 9 to the conjugation buffer in the fourth flask, wherein said antigen differs from the antigen used in step e);
h) preparing and adding oligonucleotides labelled BSA aqueous solution to the first flask;
i) preparing and adding oligonucleotides labelled BSA aqueous solution to the second flask, wherein said oligonucleotides differ from the oligonucleotides used in step h);
j) preparing and adding oligonucleotides labelled BSA aqueous solution to the third flask, wherein said oligonucleotides are complementary to the oligonucleotides used in step h);
k) preparing and adding oligonucleotides labelled BSA aqueous solution to the fourth flask, wherein said oligonucleotides are complementary to the oligonucleotides used in step i);
l) adding colloidal gold solution into each flask;
m) adding stabilizing buffer to each flask;
n) concentrating each conjugate;
o) mixing conjugates of the first and second flask; adding a surfactant and soaking a glass fibre sheet conjugate pad into the conjugate;
p) mixing conjugates of the third and fourth flask; soaking another glass fibre sheet conjugate pad into the conjugate;
q) printing capturing/sample and control lines onto the membrane,
r) laminating cards; and
s) cutting cards into strips.
or comprising the steps of
a) preparing a colloidal gold solution;
b) preparing a conjugation buffer;
c) partitioning the conjugation buffer by dividing it into a first, second, third and fourth flask;
d) adding an antibody according to claim 8 to the conjugation buffer in the first flask;
e) adding an antigen according to claim 9 to the conjugation buffer in the second flask;
f) adding an antibody according to claim 8 to the conjugation buffer in the third flask, wherein said antibody differs from the antibody used in step d);
g) adding an antigen according to claim 9 to the conjugation buffer in the fourth flask, wherein said antigen differs from the antigen used in step e);
h) preparing and adding aqueous solution comprising antibodies or antigens to the first flask, wherein said antibodies or antigens are different from the antibodies or antigens used in step d), e), g) and f);
i) preparing and adding aqueous solution comprising antibodies or antigens to the second flask, wherein said antibodies or antigens are different from the antibodies or antigens used in step d), e), g) and f) and of the antibodies used in step h);
j) preparing and adding aqueous solution comprising antibodies or antigens to the third flask, wherein said antibodies or antigens are complementary to the antibodies or antigens used in step h);
k) preparing and adding aqueous solution comprising antibodies or antigens to the fourth flask, wherein said antibodies or antigens are complementary to the antibodies or antigens used in step i);
l) adding colloidal gold solution into each flask;
m) adding stabilizing buffer to each flask;
n) concentrating each conjugate;
o) mixing conjugates of the first and second flask; adding a surfactant and soaking a glass fibre sheet conjugate pad into the conjugate;
p) mixing conjugates of the third and fourth flask; soaking another glass fibre sheet conjugate pad into the conjugate;
q) printing capturing/sample and control lines onto the membrane;
r) laminating cards; and
s) cutting cards into strips.

In another aspect the present invention relates to the use of a device according to the present invention for the detection of more than one disease in at least one sample.

In one preferred embodiment of the use of the device according to the present invention preferably two diseases are detected at the same time in the same sample.

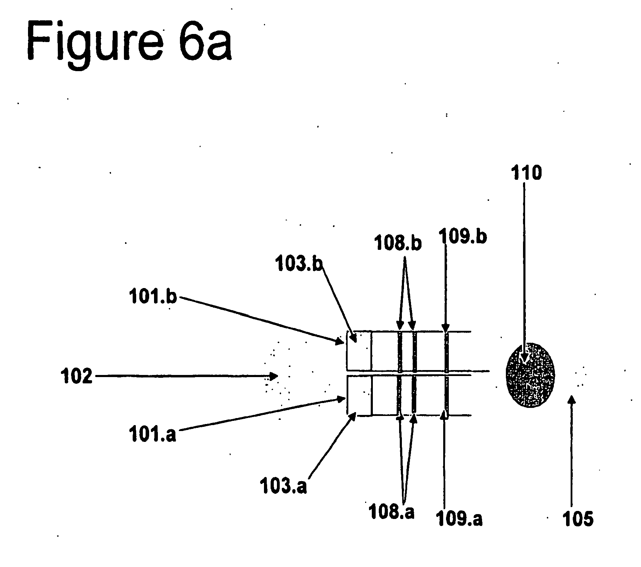

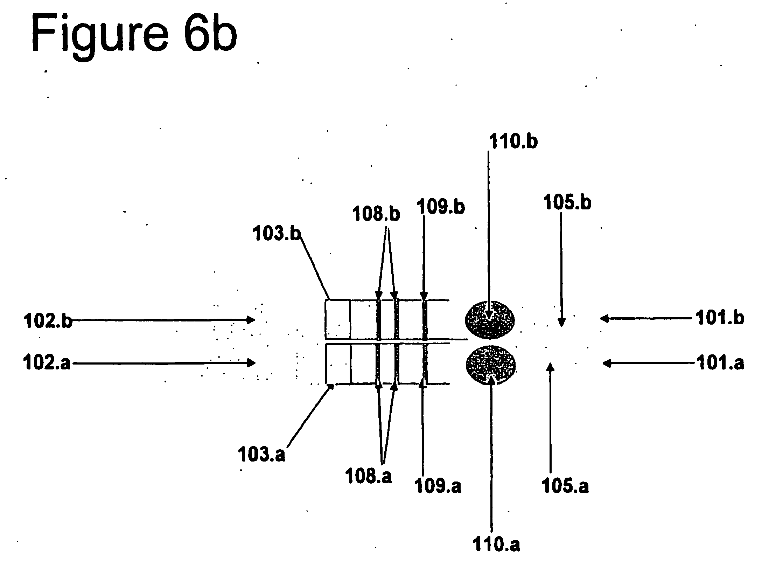

In one preferred embodiment of the use according to the present invention the antibody in one sample (e.g. specimen) and the antigen in another sample (e.g. specimen) is detected. For example, in the case two test according to the present invention are used, Lipoarabinomannan-antigen can be detected in urine, while anti-lipoarabinomannan is detected in serum (figures 6 a and b).

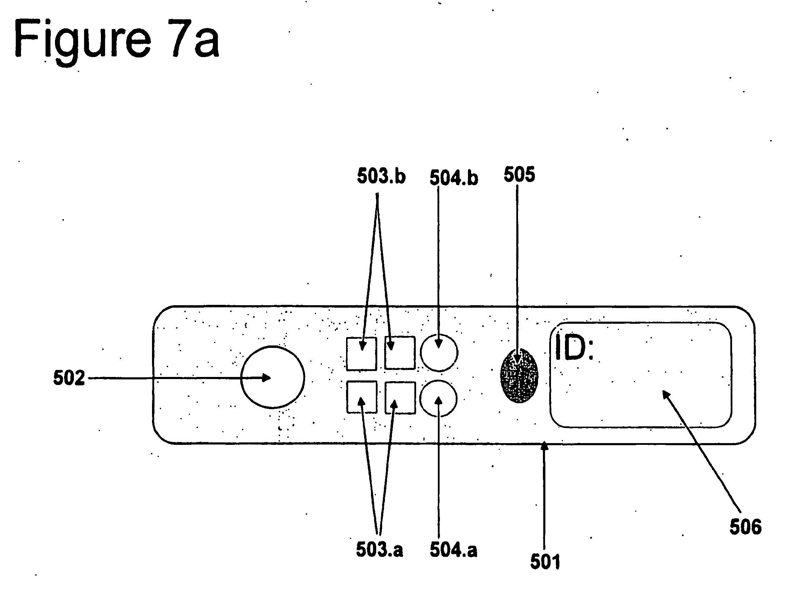

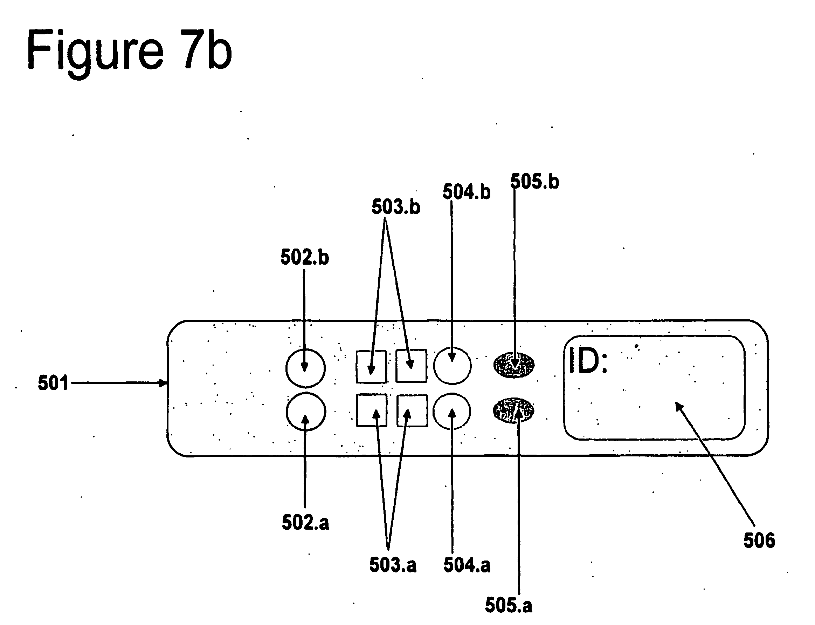

In another preferred embodiment of the use according to the present invention the antibody and antigen are detected in the same sample (specimen). For example, HIV antibodies and the HIV p24 antigen are detected in the same serum sample (specimen) using a device of two different strips (figures 7 a and b).

In another embodiment of the use of the device according to the present invention the sample was obtained from a human.

In one preferred embodiment of the use of the device according to the present invention the sample is selected from the group comprising of whole blood, serum, plasma, saliva, and urine. Preferably the sample is urine.

In another preferred embodiment of the use of the device according to the present invention the disease detected in said sample is selected from the group consisting of HIV, Hepatitis A, Hepatitis B, Hepatitis C, H. pylori, Leishmania, Schistosomiasis, Malaria, Pneumonia, Toxoplasmosis, Tubercolosis and Chlamydial infection.

In a further aspect the present invention refers to a kit for detection of a disease comprising the device according to the present invention and a manual.

In one preferred embodiment of the kit according to the present invention the kit further comprises an assay buffer. The assay buffer can be any buffer known in the art suitable for the use of whole blood samples. Preferably in the case of whole blood samples Tris buffer is used, more preferably 0.1M Tris buffer having a pH of 7.5 and comprising a preservative. Any preservative known by a person skilled in the art can be used, preferably sodium azide and even more preferably 0.01 M sodium azide is used.

The following example illustrate the present invention without, however, limiting the same thereto.

### BRIEF DESCRIPTION OF THE DRAWING

**Figure 1a****:** shows top and side views of a typical rapid-flow immunochromatographic test device known in the art in the form of a test strip 101 comprising a sample pad 102, a conjugate pad 103, a membrane 104, an absorbent pad 105, an adhesive 106, a supporting backing 107, a test zone 108, and a control zone 109.
**Figure 1b****:** shows top and side views of a preferred embodiment of a rapid-flow immunochromatographic test device according to the present invention in the form of a test strip 101 comprising a sample pad 102, a first conjugate pad 103.1, a second conjugate pad 103.2, a membrane 104, an absorbent pad 105, an adhesive 106, a supporting backing 107, two test zones 108a and 108b, a control zone 109, and the conjugates divider 110.
**Figure 2a****:** shows the schematically view of a preferred embodiment of the colloidal gold conjugates I to IV according to the present invention, wherein gold I is conjugated with an specific antibody 205 suitable to detect the target from one side (A) and two different oligonucleotides 209 and 210, gold II is conjugated with a specific antigen 206 and two different oligonucleotides 211 and 212, gold III is conjugated with another specific antibody 208 suitable to detect the antigen from the other side (B) and two different oligonucleotides 209' and 210', and gold IV is conjugated with another antigen 207 and two different oligonucleotides. The oligonucleotides of gold I and II are complementary to the oligonucleotides of gold III and IV, respectively.
**Figure 2b****:** shows the schematically view of the sample 213 that contains the two targets; two distinguish sites of the antibody 206' and 207' and that of the antigen's 205' and 208'.

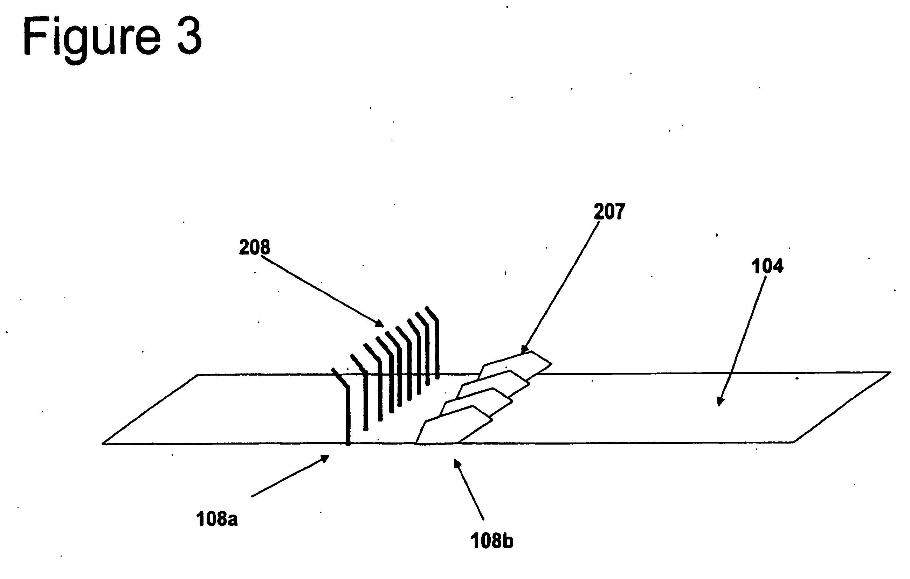

**Figure 3****:** shows a simplified scheme of a preferred embodiment of the test device according to the present invention. It shows the enlarged top view of the two test zones 108a and 108b of a test strip, whereas the second specific antibody 208 and the second specific antigen 207 are immobilized on the membrane 104.
**Figure 4****:** shows the main principle of a preferred embodiment of the signal development according to the present invention. By the sample flow within the rapid immunochromatographic test the targets in the sample will be captured by the antibody or antigen of the colloidal gold I or II to form the complexes "target-gold conjugate I" and "target-gold conjugate II". These complexes flow to the two test zones 108a and 108b, where they will be captured by a specific antibody or antigen that is immobilized onto the membrane 104 of both test zones to form a sandwich detection.
**Figure 5****:** shows the main principle of a preferred embodiment of the signal amplification and multiplication according to the present invention. After the complexes "target-gold conjugate I" and "target-gold conjugate II" were captured within the two test zones 108a and 108b, the second gold conjugate releasing pad will release the gold conjugates III and IV with the second specific antibody or antigen to capture the target analyte from the second site (let us consider it as site B) and at the same time with the complementary oligonucleotides (O-3 and O-4) that are conjugated with the first colloidal gold conjugate. The last mentioned conjugate would bind with the first conjugate from different sites, this binding could be happened between any of the conjugated oligonucleotides with its complementary oligonucleotide on the other gold conjugate or between any free site B of the analyte with its specific antibody or antigen on the conjugate.
**Figure 6a****:** shows an internal view of a preferred embodiment of a test device according to the present invention suitable to detect two different targets within the same sample (specimen) from the same person (patient). In this preferred embodiment the device comprises two test strips 101.a and 101.b, wherein both test strips share the same sample pad 102, the same humidity indicator 110 and the same absorbent pad 105. Further each test strip 101.a and 101.b comprises its own gold conjugate 103.a and 103.b, sample line 108.a and 103.b and control line 109.a and 109.b.
**Figure 6b****:** shows an internal view of a preferred embodiment of a test device according to the present invention suitable to detect two different targets within two different samples (specimens) from the same person (patient). In this preferred embodiment the device comprises two test strips 101.a and 101.b, wherein each of these two strips comprises its own sample pad 102.a and 102.b, gold conjugate 103.a and 103.b, sample line 108.a and 103.b, control line 109.a and 109.b, humidity indicator 110.a and 110.b, and absorbent pad 105.a and 105.b.
**Figure 7a****:** shows an external view of a preferred embodiment of a test device 501 according to the present invention suitable to detect two targets within the same sample (specimen) from the same person (patient). In this preferred embodiment the device 501 comprises a sample application window 502, two test result windows 503.a and 503.b, two control result windows 504.a and 504.b, humidity indication window 505 and a patient ID area 506.
**Figure 7b****:** shows an external view of a preferred embodiment of a test device 501 according to the present invention suitable to detect two targets within two different samples (specimens) from the same person (patient). In this preferred embodiment the device 501 comprises two sample application windows 502.a and 502.b, two test result windows 503.a and 503.b, two control result windows 504.a and 504.b, two humidity indication windows 505.a and 505.b and a patient ID area 506.

### EXAMPLES

### Example 1: Preparation of oligonucleotide- and complementary oligonucleotide labeled bovine serum albumin:

5mg of bovine serum albumin (BSA) was linked to each oligonucleotide and another 5mg to the complementary oligonucleotide. Every oligonucleotide had a length of about 20 nucleotides having an amino group at the 5' terminus. The procedure was performed according to the method described by Duncan et al. 1983 ²⁰comprising the following steps:

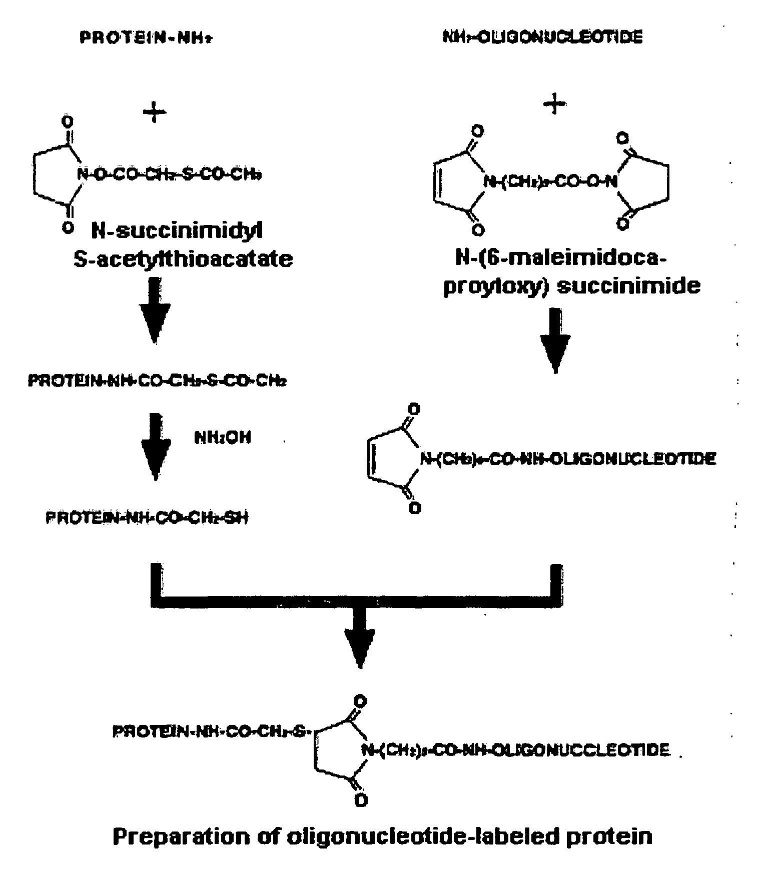

### Example 2: Preparation of an preferred embodiment of a test device according to the present invention

The oligonucleotide and complementary oligonucleotide linked BSA prepared as described in Example 1 are further processed according to a procedure comprising the following steps:
a) prepare oligonucleotide linked BSA solution, according to example 1 (solution 1);
b) prepare oligonucleotide linked BSA solution, according to example 1 (solution 2), wherein the oligonucleotides of solution 2 differs from the oligonucleotides of solution 1;
c) prepare oligonucleotide linked BSA solution, according to example 1 (solution 3), wherein the oligonucleotides of solution 3 are complementary to the oligonucleotides of solution 1;
d) prepare oligonucleotide linked BSA solution, according to example 1 (solution 4), wherein the oligonucleotides of solution 4 are complementary to the oligonucleotides of solution 2;
e) prepare 1% aqueous solution of tetrachloroauric acid at room temperature;
f) prepare 4% trisodium citrate aqueous solution at room temperature;
g) prepare 0.05 M Potassium Carbonate aqueous solution at room temperature;
h) prepare 1000ml of phosphate stabilizing buffer of pH 7.4, containing BSA, Tween 20, Sucrose, polyvinylpurrolidone and a preservative, e.g. sodium azide, at room temperature;
i) prepare colloidal gold solution by reduction of 3.4 ml boiling tetrachloroauric acid solution (after dilution into 200ml) using 2ml trisodium citrate solution and let it takes the room temperature;
j) dilute the colloidal gold solution as 1:1 using distilled water. Adjust the pH to 7.4 using potassium carbonate solution at room temperature;
k) prepare 400ml of phosphate conjugation buffer of pH 7.4 at room temperature;
l) partition the 400ml conjugation buffer by dividing it into four flasks (100ml of each);
m) add 0.5 mg of aqueous antibody (e.g. anti-Lipoarabinomannan 1^{st} clone) to the conjugation buffer in the first flask with stirring at room temperature;
n) add 0.5 mg of oligonucleotides labelled BSA aqueous solution (solution 1) to the first flask at room temperature;
o) add 0.5 mg of aqueous antigen (e.g. HIV p160) to the conjugation buffer in the second flask with stirring at room temperature;
p) add 0.5 mg of oligonucleotides labelled BSA aqueous solution (solution 2) to the second flask with stirring at room temperature;
q) add 0.5 mg of aqueous antibody (e.g. anti-Lipoarabinomannan 2^{nd} clone) to the conjugation buffer in the third flask with stirring at room temperature;
r) add 0.5 mg of oligonucleotides labelled BSA aqueous solution (solution 3) to the third flask at room temperature;
s) add 0.5 mg of aqueous antigen (e.g. HIV p160) to the conjugation buffer in the fourth flask with stirring at room temperature;
t) add 0.5 mg of oligonucleotides labelled BSA aqueous solution (solution 4) to the second flask with stirring at room temperature;
u) add 100ml colloidal gold solution into each flask with stirring at room temperature;
v) after about 45 minutes; add 200ml of stabilizing buffer to each flask;
w) after about 20 minutes; concentrate each conjugate by cooled (temperature around 15°C) high speed centrifugation (10,000 rpm for one hour);
x) discard the supernatant and re-suspend the concentrated conjugates using the stabilising buffer at room temperature;
y) adjust the concentration for each of the two conjugates to O.D.₅₂₀=2.0;
z) mix conjugates of flasks I and II, add 0.2ml of Tween 20 and soak glass fibre sheet conjugate pad into the conjugate, then heat dry at temperature around 50°C (first gold conjugate).
aa) mix conjugates of flasks III and IV, soak another glass fiber sheet conjugate pad into the conjugate, then heat dry at temperature around 50°C (second gold conjugate).
(* In case of antibodies/antigens and their specific antigens/antibodies there is no need for these steps of bovine serum albumin or any other protein labelling. ** Other proteins or peptides could be used other than bovine serum albumin).

Additionally, print first sample line (e.g. anti-HIV p24, 2^{nd} clone), second sample line (e.g. HIV p160) and control line (e.g. mixture of goat anti-p160 and goat anti-mouse IgG) onto nitrocellulose membrane, then heat dry at temperature around 50°C.

Finally, laminate cards according to the following procedure:

### A. In case of conjugate releasing site laminated within the upper side of the device plastic housing

Lamination of cards using the first gold conjugate. Laminate card components onto the backing material with the sequence:
1. laminate the nitrocellulose membrane nearly in the middle of the card;
2. laminate the absorbent pad in the end of the card (overlaps from the nitrocellulose membrane side);
3. laminate the first conjugate pad in the other side of the nitrocellulose membrane; and
4. laminate the sample pad.

### B. In case of conjugate releasing site laminated onto the test strip itself separated from the first conjugate by a divider

Laminate card components onto the backing material with the sequence (see figure 1b):
1. laminate the nitrocellulose membrane nearly in the middle of the card;
2. laminate the absorbent pad in the end of the card (overlaps from the nitrocellulose membrane side);
3. laminate the first conjugate pad in the other side of the nitrocellulose membrane;
4. laminate the plastic divider onto the first conjugate (overlaps from the nitrocellulose membrane side);
5. laminate the second conjugate pad onto the divider (overlaps from the nitrocellulose membrane side);
6. laminate the sample pad onto the other end of the card, the sample pad will overlaps with the two conjugate pads; and
7. then cut cards into strips.

### C. Alternatively

Lamination of the second gold conjugate could be applied within the plastic housing itself to ensure that the two conjugates will not propagate before release from the releasing pad and so stick within the releasing pad.

### Example 3: Ultra-sensitive urinary HIV antibody and Tuberculosis antigen Detection test

The 1 st gold conjugate pad contains:
- Gold labeled mouse anti-LAM clone 1 and at the same time the same colloidal gold is conjugated with oligonucleotides O-1.
- Gold labeled mouse anti-hlgG and at the same time the colloidal gold is conjugated with oligonucleotides O-2.

The 2nd gold conjugate pad contains:
- Gold labeled anti-LAM clone 2 and at the same time the colloidal gold is conjugated with oligonucleotides O-3 complementary to oligonucleotides O-1.
- Gold labeled recombinant envelop protein gp160 and at the same time the colloidal gold is conjugated with oligonucleotides O-4 complementary to oligonucleotides O-2.

The 1st sample line 108a (tuberculosis specific detection line) comprises anti-LAM clone 2 immobilized onto the nitrocellulose membrane. The second sample line 108b (HIV specific detection line) comprises of recombinant envelop protein gp160 immobilized onto the nitrocellulose membrane. The control line 109 is anti-mouse IgG. Sample line 108a and control line 109 turn into purple color in case of LAM availability in the sample, sample line 108b and control line 109 turn into purple color in case of HIV gp160 antibody availability in the sample, and only the control line 109 turns into purple color in case of HIV antibody and LAM antigen free sample.

There aren't any commercially available rapid tests for urinary HIV / Tuberculosis detection in urine.

### Example: 4 Ultra-sensitive urinary Hepatitis C (HCV) antibody and Tuberculosis antigen Detection test.

The 1 st gold conjugate pad contains:
- Gold labeled mouse anti-LAM clone 1 and at the same time the same colloidal gold is conjugated with oligonucleotides O-1.
- Gold labeled mouse anti-hlgG and at the same time the colloidal gold is conjugated with oligonucleotides O-2.

The 2nd gold conjugate pad contains:
- Gold labeled anti-LAM clone 2 and at the same time the colloidal gold is conjugated with oligonucleotides O-3 complementary to the oligonucleotides O-1.
- Gold labeled recombinant HCV antigen & at the same time the colloidal gold is conjugated with oligonucleotides O-4 complementary to the oligonucleotides O-2.

The 1 st sample line 108a (tuberculosis specific detection line) comprises of anti-LAM clone 2 immobilized onto the nitrocellulose membrane. The second sample line 108b (HCV specific detection line) comprises of recombinant HCV antigen immobilized onto the nitrocellulose membrane. The control line 109 is anti-mouse IgG. Sample line 108a and control line 109 turn into purple color in case of LAM availability in the sample, sample line 108b and control line 109 turn into purple color in case of HCV antibody availability in the sample, and only the control line 109 turns into purple color in case of HCV antibody and LAM antigen free sample.

There aren't any commercially available rapid tests for urinary HCV / Tuberculosis detection in urine.

### Example 5: Ultra-sensitive urinary H.Pylori antibody and Tuberculosis antigen Detection test.

The 1st gold conjugate pad contains:
- Gold labeled mouse anti-LAM clone 1 and at the same time the same colloidal gold is conjugated with oligonucleotides O-1.
- Gold labeled mouse anti-hlgG and at the same time the colloidal gold is conjugated with oligonucleotides O-2.

The 2nd gold conjugate pad contains:
- Gold labeled anti-LAM clone 2 and at the same time the colloidal gold is conjugated with oligonucleotides O-3 complementary to the oligonucleotides O-1.
- Gold labeled recombinant H.Pylori antigen and at the same time the colloidal gold is conjugated with oligonucleotides O-4 complementary to the oligonucleotides O-2.

The 1st sample line 108a (tuberculosis specific detection line) comprises of anti-LAM clone 2 immobilized onto the nitrocellulose membrane. The second sample line 108b (H.Pylori specific detection line) comprises of recombinant H.Pylori antigen immobilized onto the nitrocellulose membrane. The control line 109 is anti-mouse IgG. Sample line 108a and control line 109 turn into purple color in case of LAM availability in the sample, sample line 108b and control line 109 turn into purple color in case of H.Pylori antibody availability in the sample, and only the control line 109 turns into purple color in case of H.Pylori antibody and LAM antigen free sample.

There aren't any commercially available rapid tests for urinary H.Pylori / Tuberculosis detection in urine.

### Example 6: Other combinations are applicable using the same method like:

a. Urinary HIV/HCV/Tuberculosis Detection test.
   The 1st gold conjugate pad contains:
   - Gold labeled mouse anti-LAM clone 1 and at the same time the same colloidal gold is conjugated with oligonucleotides O-1.
   - Gold labeled mouse anti-hlgG and at the same time the colloidal gold is conjugated with oligonucleotides O-2.
   The 2nd gold conjugate pad contains:
   - Gold labeled anti-LAM clone 2 and at the same time the colloidal gold is conjugated with oligonucleotides O-3 complementary to the oligonucleotides O-1.
   - Gold labeled recombinant Hepatitis C antigen and at the same time the colloidal gold is conjugated with oligonucleotides O-4 complementary to the oligonucleotides O-2.
   - Gold labeled recombinant envelop protein gp160 and at the same time the colloidal gold is conjugated with oligonucleotides O-4 complementary to the oligonucleotides O-2.
   The 1st sample line 108a (tuberculosis specific detection line) comprises of anti-LAM clone 2 immobilized onto the nitrocellulose membrane. The second sample line 108b (HCV specific detection line) comprises of recombinant HCV antigen immobilized onto the nitrocellulose membrane. The third sample line 108c (HIV specific detection line) comprises of recombinant envelop protein gp160 immobilized onto the nitrocellulose membrane. The control line 109 is anti-mouse IgG. Sample line 108a and control line 109 turn into purple color in case of LAM availability in the sample, sample line 108b and control line109 turn into purple color in case of HCV antibody availability in the sample, sample line 108c and control line109 turn into purple color in case of HIV antibody availability in the sample and only the control line 109 turns into purple color in case of HCV antibodies, HIV antibodies and LAM antigen free sample.
b. Urinary Tuberculosis antigen, Pneumonia antigen and H.Pylori antibody Detection test.
   The 1st gold conjugate pad contains:
   - Gold labeled mouse anti-LAM clone 1 and at the same time the same colloidal gold is conjugated with oligonucleotides O-1.
   - Gold labeled mouse anti-hlgG and at the same time the colloidal gold is conjugated with oligonucleotides O-2.
   The 2nd gold conjugate pad contains:
   - Gold labeled anti-LAM clone 2 and at the same time the colloidal gold is conjugated with oligonucleotides O-3 complementary to the oligonucleotides O-1.
   - Gold labeled recombinant Hepatitis C antigen and at the same time the colloidal gold is conjugated with oligonucleotides O-4 complementary to the oligonucleotides O-2.
   - Gold labeled recombinant envelop protein gp160 and at the same time the colloidal gold is conjugated with oligonucleotides O-4 complementary to the oligonucleotides O-2.
   The 1st sample line 108a (tuberculosis specific detection line) comprises of anti-LAM clone 2 immobilized onto the nitrocellulose membrane. The second sample line 108b (Pneumonia specific detection line) comprises of anti-pneumolysin (PLY) clone 2 immobilized onto the nitrocellulose membrane. The third sample line 108c (H.Pylori specific detection line) comprises of recombinant H.Pylori antigen immobilized onto the nitrocellulose membrane. The control line 109 is anti-mouse IgG. Sample line 108a and control line 109 turn into purple color in case of LAM availability in the sample, sample line 108b and control line109 turn into purple color in case of PLY antibody availability in the sample, sample line 108c and control line109 turn into purple color in case of H.Pylori antibodies availability in the sample and only the control line 109 turns into purple color in case of H.Pylori antibodies, PLY and LAM antigen free sample.
c. Urinary HIV/HCV/Malaria Detection test.
   The 1st gold conjugate pad contains:
   - Gold labeled mouse anti-HRP-II clone 1 and at the same time the same colloidal gold is conjugated with oligonucleotides O-1.
   - Gold labeled mouse anti-hlgG and at the same time the colloidal gold is conjugated with oligonucleotides O-2.
   The 2nd gold conjugate pad contains:
   - Gold labeled anti- HRP-11 clone 2 and at the same time the colloidal gold is conjugated with oligonucleotides O-3 complementary to the oligonucleotides O-1.
   - Gold labeled recombinant Hepatitis C antigen and at the same time the colloidal gold is conjugated with oligonucleotides O-4 complementary to the oligonucleotides O-2.
   - Gold labeled recombinant envelop protein gp160 and at the same time the colloidal gold is conjugated with oligonucleotides O-4 complementary to the oligonucleotides O-2.
   The 1st sample line 108a (malaria specific detection line) comprises of anti-HRP-II clone 2 immobilized onto the nitrocellulose membrane. The second sample line 108b (HCV specific detection line) comprises of recombinant HCV antigen immobilized onto the nitrocellulose membrane. The third sample line 108c (HIV specific detection line) comprises of recombinant envelop protein gp160 immobilized onto the nitrocellulose membrane. The control line 109 is anti-mouse IgG. Sample line 108a and control line 109 turn into purple color in case of HRP-II availability in the sample, sample line 108b and control line 109 turn into purple color in case of HCV antibody availability in the sample, sample line 108c and control line109 turn into purple color in case of HIV antibody availability in the sample and only the control line 109 turns into purple color in case of HCV antibodies, HIV antibodies and HRP-II antigen free sample.
d. Urinary HIV/HCV/Malaria/Tuberculosis Detection test.
e. Urinary HIV antibody and Malaria antigen Detection test.
f. Urinary HIV antibody, Malaria antigen and Tuberculosis Antigen Detection test.
g. Urinary HCV antibody and Malaria antigen Detection test.

### References

(1) J Chandler, N Robinson, and K Whiting, "Handling False Signals in Gold-Based Rapid Tests", IVD Technology 7, no. 2 (2001): 34-45; http://www.devicelink.com/ivdt/archive/01/03/002.html.
(2) J Chandler, T Gurmin, and N Robinson, "The Place of Gold in Rapid Tests", IVD Technology 6, no. 2 (2000): 37-49; http://www.devicelink.com/ivdt/archive/00/03/004.html
(3) TC Tisone et al., "Image Analysis for Rapid-Flow Diagnostics", IVD Technology 5, no. 5 (1999): 52-58; http://www.devicelink.com/ivdt/archive/99/09/010.html.
(4) Zaaijer, H.L., Exel-Oehlers, P.V., Kraaijeveld, T., Altena, E., Lelie, P.N. (1992) Early detection of antibodies to HIV-1 by third-generation assays. Lancet 340, 770-772.
(5) Constantine, N.T., van der Groen, G., Belsey, E.M., Tamashiro, H. (1994) Sensitivity of HIV-antibody assays determined by seroconversion panels. AIDS 8, 1715-1720.
(6) Satten, G.A., Busch, M.P., et al. (1997) Effect of transmission route on window period estimates. Fourth Conference on Retroviruses and Opportunistic Infections, Washington DC, Abstract 122.
(7) WHO. Rapid HIV tests: Guidelines for use in HIV testing and counseling services in resource-constrained settings. Geneva 2004. http://www.who.int/hiv/pub/vct/rapidhivtests/en/
(8) Holodniy M, et al. (1991) Reduction in plasma human immunodeficiency virus ribonucleic acid following dideoxynucleoside therapy as determined by the polymerase chain reaction. J. Clin. Invest 88, 1755-1759.
(9) Katzenstein D.A., et al. (1994) Quantitation of human immunodeficiency virus by culture and polymerase chain reaction in response to didanosine after long-term therapy with zidovudine. J. Infect. Dis. 169, 416-419.
(10) Jackson JB, et al. (1998) Practical diagnostic testing for human immunodeficiency virus. Clin. Microb. Rev. 1, 124-138.
(11) Goudsmit J, et al. (1986) Expression of human Immunodeficiency virus antigen (HIV-Ag) in serum and cerebrospinal fluid during acute and chronic infection. Lancet 2, 177-180.
(12) Aubuchon, J.P., Birkmeyer, J.D., Busch, M.P. (1997) Cost-effectiveness of expanded human immunodeficiency virus-testing protocols for donated blood. Transfusion 45, 45-51.
(13) Ward, J.M., Holmberg, S.D., Allen, J.R., Cohn, D.L., et al. (1988) Transmission of human immunodeficiency virus (HIV) by blood transfusion screened as negative for HTV antibody. N. Engl. J. Med 8, 473-478.
(14) Alter, H.J., et al. (1990) Prevalence of human immunodeficiency virus type 1 p24 antigen in U.S. blood donors - an assessment of the efficacy of testing in donor screening. N. Engl. J. Med 323, 1312-1317.
(15) Mayers, D.L. (1998) Drug-resistant HIV-1. The virus strikes back. JAMA 279, 2000-2002.
(16) Stephenson, J. (2002) Cheaper HIV drugs for poor nations bring a new challenge: monitoring treatment. JAMA 288, 2.
(17) WHO. HIV assays: Operational characteristics (Phase 1). Report 15/ antigen/antibody ELISAs. Geneva 2004. http://www.who.int/diagnostics_laboratory/evaluations/hiv/en/
(18) WHO. HIV assays: Operational characteristics (Phase 1). Report 14/ simple/rapid tests. Geneva 2003. http://www.who.int/diagnostics_laboratory/evaluations/hiv/en/
(19) Meier, T, et al. (2001) Evidence for a diagnostic window in fourth generation assays for HIV. J. Clin. Virol. 23, 113-116.
(20) Duncan, R.J.S., Weston, P.D., Wrigglesworth, R., 1983. A new reagent which may be used to introduce sulfhydryl groups into proteins, and its use in the preparation of conjugates for immunoassay. Anal. Biochem. 132, 68.
(21) R Tanaka, T Yuhi, N Nagatani, T Endo, K Kerman, Y Takamura, E Tamiya (2006) A novel enhancement assay for immunochromatographic test strips using gold nanoparticles. Anal Bioanal Chem 385: 1414-1420.
(22) Hazarika, P., Ceyhan, B., Niemeyer, C. M. (2005) Sensitive detection of proteins using difunctional DNA-gold nanoparticles. Small 1, 844-848.

## Claims

1. A rapid immunochromatographic test device for the detection of more than one target in a sample, comprising
(a) a first gold conjugate releasing pad, comprising at least two colloidal gold conjugates I and II, wherein colloidal gold conjugate I is conjugated with a first antibody capturing a target antigen from a first site and at least one oligonucleotide, and wherein colloidal gold conjugate II is conjugated with a first antigen being captured by a target antibody or second antibody being specific for said target antibody and at least one oligonucleotide, wherein the oligonucleotide of colloidal gold conjugate II differs from the oligonucleotide of colloidal gold conjugate I;
wherein the colloidal gold conjugates I and II form "target-gold conjugate I" and "target-gold conjugate II";
(b) a second gold conjugate releasing pad, comprising at least two colloidal gold conjugates III and IV, wherein colloidal gold conjugate III is conjugated with a third antibody capturing the target antigen from a second site and at least one oligonucleotide, wherein the oligonucleotide of colloidal gold conjugate III is complementary to the oligonucleotide of colloidal gold conjugate I, and wherein colloidal gold conjugate IV is conjugated with a second antigen being captured by the same target antibody that captures the first antigen or fourth antibody being specific for said target antibody and at least one oligonucleotide, wherein the oligonucleotide of colloidal gold conjugate IV is complementary to the oligonucleotide of colloidal gold conjugate II;
wherein both gold conjugate releasing pads are located at different positions within the test device;
(c) a test strip comprising a sample pad, a conjugate pad comprising said first gold conjugate releasing pad, a conjugate pad comprising said second gold conjugate releasing pad, a membrane comprising two capture test zones and a negative control zone, and an absorbent pad;
wherein one capture test zone comprises said third antibody and wherein the other capture test zone comprises said second antigen or fourth antibody;
such that after the "target-gold conjugate I" and "target-gold conjugate II" are captured within the two capture test zones, the second gold conjugate releasing pad will release the colloidal gold conjugates III and IV.

2. A rapid immunochromatographic test device for the detection of more than one target in a sample, comprising
(a) a first gold conjugate releasing pad, comprising at least two colloidal gold conjugates I and II, wherein colloidal gold conjugate I is conjugated with a first antibody capturing a target antigen from a first site and at least one further antibody or antigen (A-1), and wherein colloidal gold conjugate II is conjugated with a first antigen being captured by a target antibody or second antibody being specific for said target antibody and at least one further antibody or antigen (A-2), wherein said antibodies or antigens (A-1) and (A-2) differ from each other and differ from the first and second antigens and antibodies;
wherein the colloidal gold conjugates I and II form. "target-gold conjugate I" and "target-gold conjugate II";
(b) a second gold conjugate releasing pad, comprising at least two colloidal gold conjugates III and IV, wherein colloidal gold conjugate III is conjugated with a third antibody capturing the target antigen from a second site and at least one further antibody or antigen (A-3), wherein said antibody or antigen (A-3) is complementary to said antibody or antigen (A-1), and wherein colloidal gold conjugate IV is conjugated with a second antigen being captured by the same target antibody that captures the first antigen or fourth antibody being specific for said target antibody and at least one further antibody or antigen (A-4), wherein said antibody or antigen (A-4) is complementary to said antibody or antigen (A-2);
wherein both gold conjugate releasing pads are located at different positions within the test device.
(c) a test strip comprising a sample pad, a conjugate pad comprising said first gold conjugate releasing pad, a conjugate pad comprising said second gold conjugate releasing pad, a membrane comprising two capture test zones and a negative control zone, and an absorbent pad;
wherein one capture test zone comprises said third antibody and wherein the other capture test zone comprises said second antigen or fourth antibody;
such that after the "target-gold conjugate I" and "target-gold conjugate II" are captured within the two capture test zones, the second gold conjugate releasing pad will release the colloidal gold conjugates III and IV.

3. The device according to claim 1 or 2, wherein said membrane is attached by means of an adhesive to a supporting backing.

4. The device according to any of claims 1 to 3, wherein said first and second gold conjugate pad are laminated between the sample pad and the membrane, wherein said two gold conjugates are separated by a divider.

5. The device according to any of claims 1 to 3, wherein said first gold conjugate pad is attached between the sample pad and the membrane while the second gold conjugate pad is within the upper part of the plastic housing.

6. The device according to any of claims 3 to 5, wherein said supporting backing is a plastic backing.

7. The device according to any of the preceding claims, wherein said membrane is nitrocellulose membrane.

8. The device according to any of claims 1 to 7, wherein said first and third antibody is selected from the group comprising mouse anti-HIV p24, mouse anti-HBsAg, anti-hlgG, anti-Lipoarabinomannan, anti-H. pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

9. The device according to any of claims 1 to 7, wherein said first and second antigen is selected from the group comprising conjugate of HIV antigen, conjugate of Hepatitis C antigen, HIV I antigen (HIV p160), HIV 2 antigen (HIV p36), Hepatitis B antigen, Lipoarabinomannan, H. pylori antigen, Toxoplasma antigen.

10. The device according to any of the preceding claims, wherein said control zone comprises non-specific capturing antibody and/or a non-specific antibody capturing protein.

11. The device according to claim 10, wherein said non-specific antibody is selected from the group consisting of anti-mouse IgG, anti-rabbit IgG, anti-goat IgG, anti-donkey IgG, Anti-sheep IgG, anti-HN p24, anti-Lipoarabinomannan, anti-H. pylori antigen, anti-Leishmania antigen, anti-Pneumonia antigen, anti-Malaria antigen, anti-Chlamydia antigen, anti-Toxoplasma antigen, anti-Schistosoma antigen, HIV 1 antibody, and HIV 2 antibody.

12. The device according to claim 10, wherein said non-specific capturing protein selected is either Protein A or Protein G.

13. A method for the production of a device according to any of claims 1 and 3 to 12, comprising the steps of
a) preparing a colloidal gold solution;
b) preparing a conjugation buffer;
c) partitioning the conjugation buffer by dividing it into a first, second, third and fourth flask;
d) adding an antibody according to claim 8 to the conjugation buffer in the first flask;
e) adding an antigen according to claim 9 to the conjugation buffer in the second flask;
f) adding an antibody according to claim 8 to the conjugation buffer in the third flask, wherein said antibody differs from the antibody used in step d);
g) adding an antigen according to claim 9 to the conjugation buffer in the fourth flask, wherein said antigen differs from the antigen used in step e);
h) preparing and adding oligonucleotides labelled BSA aqueous solution to the first flask;
i) preparing and adding oligonucleotides labelled BSA aqueous solution to the second flask, wherein said oligonucleotides differ from the oligonucleotides used in step h);
j) preparing and adding oligonucleotides labelled BSA aqueous solution to the third flask, wherein said oligonucleotides are complementary to the oligonucleotides used in step h);
k) preparing and adding oligonucleotides labelled BSA aqueous solution to the fourth flask, wherein said oligonucleotides are complementary to the oligonucleotides used in step i);
l) adding colloidal gold solution into each flask;
m) adding stabilizing buffer to each flask;
n) concentrating each conjugate;
o) mixing conjugates of the first and second flask; adding a surfactant and soaking a glass fibre sheet conjugate pad into the conjugate;
p) mixing conjugates of the third and fourth flask; soaking another glass fibre sheet conjugate pad into the conjugate;
q) printing capturing/ample and control lines onto the membrane;
r) laminating cards; and
s) cutting cards into strips.

14. A method for the production of a device according to any of claims 2 to 12, comprising the steps of
a) preparing a colloidal gold solution;
b) preparing a conjugation buffer;
c) partitioning the conjugation buffer by dividing it into a first, second, third and fourth flask;
d) adding an antibody according to claim 8 to the conjugation buffer in the first flask;
e) adding an antigen according to claim 9 to the conjugation buffer in the second flask;
f) adding an antibody according to claim 8 to the conjugation buffer in the third flask, wherein said antibody differs from the antibody used in step d);
g) adding an antigen according to claim 9 to the conjugation buffer in the fourth flask, wherein said antigen differs from the antigen used in step e);
h) preparing and adding aqueous solution comprising antibodies or antigens to the first flask, wherein said antibodies or antigens are different from the antibodies or antigens used in step d), e), g) and f);
i) preparing and adding aqueous solution comprising antibodies or antigens to the second flask, wherein said antibodies or antigens are different from the antibodies or antigens used in step d), e), g) and f) and of the antibodies used in step h);
j) preparing and adding aqueous solution comprising antibodies or antigens to the third flask, wherein said antibodies or antigens are complementary to the antibodies or antigens used in step h);
k) preparing and adding aqueous solution comprising antibodies or antigens to the fourth flask, wherein said antibodies or antigens are complementary to the antibodies or antigens used in step i);
l) adding colloidal gold solution into each flask;
m) adding stabilizing buffer to each flask;
n) concentrating each conjugate;
o) mixing conjugates of the first and second flask,; adding a surfactant and soaking a glass fibre sheet conjugate pad into the conjugate;
p) mixing conjugates of the third and fourth flask; soaking another glass fibre sheet conjugate pad into the conjugate;
q) printing capturing/sample and control lines onto the membrane;
r) laminating cards; and
s) cutting cards into strips.

15. Use of a device according to any of claims 1 to 12 for the detection of at least two diseases in at least one sample.

16. The use according to claim 15, wherein two diseases are detected at the same time in one sample.

17. The use according to claim 15 or 16, wherein said sample was obtained from a human.

18. The use according to claim 15, wherein said sample is selected from the group comprising of whole blood, serum, plasma, saliva, and urine,

19. The use according to any of claims 15 to 18, wherein said disease detected in said sample is selected from the group consisting of HIV, Hepatitis A, Hepatitis B, Hepatitis C, H. Pylori, Leishmania, Schistosomiasis, Malaria, Pneumonia, Toxoplasmosis, Tuberculosis and Chlamydial infection.

20. Kit for detection of a disease comprising the device according to any of claims 1 to 12 and a manual.

21. The kit according to claim 20, further comprising an assay buffer.

22. The kit according to claim 21, wherein said assay buffer comprises a preservative.

## Patentansprüche

1. Immunochromatographische Schnelltestvorrichtung zur Detektion von mehr als einem Target in einer Probe, umfassend
(a) ein erstes Goldkonjugatfreisetzungskissen, umfassend wenigstens zwei kolloidale Goldkonjugate I und II, wobei das kolloidale Goldkonjugat I mit einem ersten Antikörper, der ein Target-Antigen von einer ersten Stelle einfängt, und wenigstens einem Oligonukleotid konjugiert ist und wobei das kolloidale Goldkonjugat II mit einem ersten Antigen, das durch einen Target-Antikörper eingefangen wird, oder zweiten Antikörper, der für den Target-Antikörper spezifisch ist, und wenigstens einem Oligonukleotid konjugiert ist, wobei das Oligonukleotid des kolloidalen Goldkonjugats II sich von dem Oligonukleotid des kolloidalen Goldkonjugats I unterscheidet;
wobei die kolloidalen Goldkonjugate I und II "Target-Goldkonjugat I" und "Target-Goldkonjugat II" bilden;
(b) ein zweites Goldkonjugatfreisetzungskissen, umfassend wenigstens zwei kolloidale Goldkonjugate III und IV, wobei das kolloidale Goldkonjugat III mit einem dritten Antikörper, der das Target-Antigen von einer zweiten Stelle einfängt, und wenigstens einem Oligonukleotid konjugiert ist, wobei das Oligonukleotid des kolloidalen Goldkonjugats III komplementär zum Oligonukleotid des kolloidalen Goldkonjugats I ist, und wobei das kolloidale Goldkonjugat IV mit einem zweiten Antigen, das durch denselben Target-Antikörper eingefangen wird, der das erste Antigen einfängt, oder vierten Antikörper, der für den Target-Antikörper spezifisch ist, und wenigstens einem Oligonukleotid konjugiert ist, wobei das Oligonukleotid des kollodialen Goldkonjugats IV zum Oligonukleotid des kolloidalen Goldkonjugats II komplementär ist;
wobei beide Goldkonjugatfreisetzungskissen an unterschiedlichen Positionen in der Testvorrichtung angeordnet sind;
(c) einen Teststreifen, umfassend ein Probenkissen, ein Konjugatkissen, umfassend das erste Goldkonjugatfreisetzungskissen, ein Konjugatkissen, umfassend das zweite Goldkonjugatfreisetzungskissen, eine Membran, umfassend zwei Einfangtestzonen und eine Negativkontrollzone, und ein Absorptionskissen;
wobei eine Einfangtestzone den dritten Antikörper umfasst und wobei die andere Einfangtestzone das zweite Antigen oder den vierten Antikörper umfasst;
so dass, nachdem das "Target-Goldkonjugat I" und "Target-Goldkonjugat II" in den zwei Einfangtestzonen eingefangen sind, das zweite Goldkonjugatfreisetzungskissen die kolloidalen Goldkonjugate III und III freisetzen wird.

2. Immunochromatographische Schnelltestvorrichtung zur Detektion von mehr als einem Target in einer Probe, umfassend
(a) ein erstes Goldkonjugatfreisetzungskissen, umfassend wenigstens zwei kolloidale Goldkonjugate I und II, wobei das kolloidale Goldkonjugat I mit einem ersten Antikörper, der ein Target-Antigen von einer ersten Stelle einfängt, und wenigstens einem weiteren Antikörper oder Antigen (A-1) konjugiert ist und wobei das kolloidale Goldkonjugat II mit einem ersten Antigen, das durch einen Target-Antikörper eingefangen wird, oder zweiten Antikörper, der für den Target-Antikörper spezifisch ist, und wenigstens einem weiteren Antikörper oder Antigen (A-2) konjugiert ist, wobei die Antikörper oder Antigene (A-1) und (A-2) sich voneinander unterscheiden und sich von den ersten und zweiten Antigenen und Antikörpern unterscheiden;
wobei die kolloidalen Goldkonjugate I und II "Target-Goldkonjugat I" und "Target-Goldkonjugat II" bilden;
(b) ein zweites Goldkonjugatfreisetzungskissen, umfassend wenigstens zwei kolloidale Goldkonjugate III und IV, wobei das kolloidale Goldkonjugat III mit einem dritten Antikörper, der das Target-Antigen von einer zweiten Stelle einfängt, und wenigstens einem weiteren Antikörper oder Antigen (A-3) konjugiert ist, wobei der Antikörper oder das Antigen (A-3) komplementär zum Antikörper oder Antigen (A-1) ist, und wobei das kolloidale Goldkonjugat IV mit einem zweiten Antigen, das durch denselben Target-Antikörper eingefangen wird, der das erste Antigen einfängt, oder vierten Antikörper, der für den Target-Antikörper spezifisch ist, und wenigstens einem weiteren Antikörper oder Antigen (A-4) konjugiert ist, wobei der Antikörper oder das Antigen (A-4) komplementär zum Antikörper oder Antigen (A-2) ist;
wobei beide Goldkonjugatfreisetzungskissen an unterschiedlichen Positionen in der Testvorrichtung angeordnet sind;
(c) einen Teststreifen, umfassend ein Probenkissen, ein Konjugatkissen, umfassend das erste Goldkonjugatfreisetzungskissen, ein Konjugatkissen, umfassend das zweite Goldkonjugatfreisetzungskissen, eine Membran, umfassend zwei Einfangtestzonen und eine Negativkontrollzone, und ein Absorptionskissen;
wobei eine Einfangtestzone den dritten Antikörper umfasst und wobei die andere Einfangtestzone das zweite Antigen oder den vierten Antikörper umfasst;
so dass, nachdem das "Target-Goldkonjugat I" und "Target-Goldkonjugat II" in den zwei Einfangtestzonen eingefangen sind, das zweite Goldkonjugatfreisetzungskissen die kolloidalen Goldkonjugate III und III freisetzen wird.

3. Vorrichtung nach Anspruch 1 oder 2, wobei die Membran mittels eines Klebers an einer Tragerückschicht befestigt ist.

4. Vorrichtung nach einem der Ansprüche 1 bis 3, wobei das erste und zweite Goldkonjugatkissen zwischen das Probenkissen und die Membran laminiert sind, wobei die zwei Goldkonjugate durch einen Teiler getrennt sind.

5. Vorrichtung nach einem der Ansprüche 1 bis 3, wobei das erste Goldkonjugatkissen zwischen dem Probenkissen und der Membran angebracht ist, während das zweite Goldkonjugatkissen sich im oberen Teil des Kunststoffgehäuses befindet.

6. Vorrichtung nach einem der Ansprüche 3 bis 5, wobei die Tragerückschicht eine Kunststoffrückschicht ist.

7. Vorrichtung nach einem der vorangehenden Ansprüche, wobei die Membran Nitrocellulose-Membran ist.

8. Vorrichtung nach einem der Ansprüche 1 bis 7, wobei der erste und dritte Antikörper ausgewählt ist aus der Gruppe, umfassend Maus-anti-HIV p24, Maus-anti-HBsAg, anti-hlgG, anti-Lipoarabinomannan, anti-H. pylori-Antigen, anti-Leishmania-Antigen, anti-Pneumonia-Antigen, anti-Malaria-Antigen, anti-Chlamydia-Antigen, anti-Toxoplasma-Antigen, anti-Schistosoma-Antigen, HIV-1-Antikörper und HIV-2-Antikörper.

9. Vorrichtung nach einem der Ansprüche 1 bis 7, wobei das erste und zweite Antigen ausgewählt ist aus der Gruppe, umfassend Konjugat von HIV-Antigen, Konjugat von Hepatitis-C-Antigen, HIV-1-Antigen (HIV p160), HIV-2-Antigen (HIV p36), Hepatitits-B-Antigen, Lipoarabinomannan, H. pylori-Antigen, Toxoplasma-Antigen.

10. Vorrichtung nach einem der vorangehenden Ansprüche, wobei die Kontrollzone einen nicht-spezifischen Einfangantikörper und/oder ein nicht-spezifisches Antikörpereinfangprotein umfasst.

11. Vorrichtung nach Anspruch 10, wobei der nicht-spezifische Antikörper ausgewählt ist aus der Gruppe, bestehend aus anti-Maus-IgG, anti-Kaninchen-IgG, anti-Ziege-IgG, anti-Esel-IgG, anti-Schaf-IgG, anti-HIV p24, anti-Lipoarabinomannan, anti-H. pylori-Antigen, anti-Leishmania-Antigen, anti-Pneumonia-Antigen, anti-Malaria-Antigen, anti-Chlamydia-Antigen, anti-Toxoplasma-Antigen, anti-Schistosoma-Antigen, HIV-1-Antikörper und HIV-2-Antikörper.

12. Vorrichtung nach Anspruch 10, wobei das nicht-spezifische Einfangprotein ausgewählt ist aus entweder Protein A oder Protein G.

13. Verfahren zur Herstellung einer Vorrichtung nach einem der Ansprüche 1 und 3 bis 12, das die Schritte umfasst
a) Herstellen einer kolloidalen Goldlösung;
b) Herstellen eines Konjugationspuffers;
c) Aufteilen des Konjugationspuffers durch Verteilen desselben in einen ersten, zweiten, dritten und vierten Kolben;
d) Zugeben eines Antikörpers nach Anspruch 8 zum Konjugationspuffer im ersten Kolben;
e) Zugeben eines Antigens nach Anspruch 9 zum Konjugationspuffer im zweiten Kolben;
f) Zugeben eines Antikörpers nach Anspruch 8 zum Konjugationspuffer im dritten Kolben, wobei der Antikörper sich von dem in Schritt d) verwendeten Antikörper unterscheidet;
g) Zugeben eines Antigens nach Anspruch 9 zum Konjugationspuffer im vierten Kolben, wobei sich das Antigen von dem in Schritt e) verwendeten Antigen unterscheidet;
h) Herstellen und Zugeben von mit Oligonukleotiden markierter wässrigen BSA-Lösung zum ersten Kolben;
i) Herstellen und Zugeben von mit Oligonukleotiden markierter wässrigem BSA-Lösung zum zweiten Kolben, wobei die Oligonukleotide sich von den in Schritt h) verwendeten Oligonukleotiden unterscheiden;
j) Herstellen und Zugeben von mit Oligonukleotiden markierter wässrigem BSA-Lösung zum dritten Kolben, wobei die Oligonukleotide zu den in Schritt h) verwendeten Oligonukleotiden komplementär sind;
k) Herstellen und Zugeben von mit Oligonukleotiden markierter wässrigem BSA-Lösung zum vierten Kolben, wobei die Oligonukleotide zu den in Schritt i) verwendeten Oligonukleotiden komplementär sind;
l) Zugeben von kolloidaler Goldlösung in jeden Kolben;
m) Zugeben von Stabilisierungspuffer zu jedem Kolben;
n) Konzentrieren jeden Konjugats;
o) Mischen der Konjugate des ersten und zweiten Kolbens; Zugeben eines Tensids und Tränken eines Glasfaserschichtkonjugatkissens im Konjugat;
p) Mischen der Konjugate des dritten und vierten Kolbens; Tränken eines weiteren Glasfaserschichtkonjugatkissens im Konjugat;
q) Drucken von Einfang/Proben- und Kontrolllinien auf die Membran;
r) Laminieren von Karten; und
s) Zuschneiden der Karten zu Streifen.

14. Verfahren zur Herstellung einer Vorrichtung nach einem der Ansprüche 2 bis 12, das die Schritte umfasst
a) Herstellen einer kolloidalen Goldlösung;
b) Herstellen eines Konjugationspuffers;
c) Aufteilen des Konjugationspuffers durch Verteilen desselben in einen ersten, zweiten, dritten und vierten Kolben;
d) Zugeben eines Antikörpers nach Anspruch 8 zum Konjugationspuffer im ersten Kolben;
e) Zugeben eines Antigens nach Anspruch 9 zum Konjugationspuffer im zweiten Kolben;
f) Zugeben eines Antikörpers nach Anspruch 8 zum Konjugationspuffer im dritten Kolben, wobei der Antikörper sich von dem in Schritt d) verwendeten Antikörper unterscheidet;
g) Zugeben eines Antigens nach Anspruch 9 zum Konjugationspuffer im vierten Kolben, wobei sich das Antigen von dem in Schritt e) verwendeten Antigen unterscheidet;
h) Herstellen und Zugeben von wässriger Lösung, die Antikörper oder Antigene umfasst, zum ersten Kolben, wobei die Antikörper oder Antigene von den in Schritt d), e), g) und f) verwendeten Antikörpern oder Antigenen verschieden sind;
i) Herstellen und Zugeben von wässriger Lösung, die Antikörper oder Antigene umfasst, zum zweiten Kolben, wobei die Antikörper oder Antigene von den in Schritt d), e), g) und f) verwendeten Antigenen und von den in Schritt h) verwendeten Antikörpern verschieden sind;
j) Herstellen und Zugeben von wässriger Lösung, die Antikörper oder Antigene umfasst, zum dritten Kolben, wobei die Antikörper oder Antigene zu den in Schritt h) verwendeten Antikörpern oder Antigenen komplementär sind;
k) Herstellen und Zugeben von wässriger Lösung, die Antikörper oder Antigene umfasst, zum vierten Kolben, wobei die Antikörper oder Antigene zu den in Schritt i) verwendeten Antikörpern oder Antigenen komplementär sind;
l) Zugeben von kolloidaler Goldlösung in jeden Kolben;
m) Zugeben von Stabilisierungspuffer zu jedem Kolben;
n) Konzentrieren jeden Konjugats;
o) Mischen der Konjugate des ersten und zweiten Kolbens; Zugeben eines Tensids und Tränken eines Glasfaserschichtkonjugatkissens im Konjugat;
p) Mischen der Konjugate des dritten und vierten Kolbens; Tränken eines weiteren Glasfaserschichtkonjugatkissens im Konjugat;
q) Drucken von Einfang/Proben- und Kontrolllinien auf die Membran;
r) Laminieren von Karten; und
s) Zuschneiden der Karten zu Streifen.

15. Verwendung der Vorrichtung nach einem der Ansprüche 1 bis 12 zur Detektion in wenigstens zwei Erkrankungen in wenigstens einer Probe.

16. Verwendung nach Anspruch 15, wobei zwei Erkrankungen gleichzeitig in einer Probe nachgewiesen werden.

17. Verwendung nach Anspruch 15 oder 16, wobei die Probe von einem Menschen erhalten wurde.

18. Verwendung nach Anspruch 15, wobei die Probe ausgewählt ist aus der Gruppe, umfassend Vollblut, Serum, Plasma, Speichel und Harn.

19. Verwendung nach einem der Ansprüche 15 bis 18, wobei die Erkrankung, die in der Probe nachgewiesen wird, ausgewählt ist aus der Gruppe, bestehend aus HIV, Hepatitis A, Hepatitis B, Hepatitis C, H. pylori, Leishmania, Schistosomiasis, Malaria, Pneumonie, Toxoplasmose, Tuberkulose und Chlamydien-Infektion.

20. Kit zur Detektion einer Erkrankung, der die Vorrichtung nach einem der Ansprüche 1 bis 12 und eine Anleitung umfasst.

21. Kit nach Anspruch 20, der weiter einen Testpuffer umfasst.

22. Kit nach Anspruch 21, wobei der Testpuffer einen Konservierungsstoff umfasst.

## Revendications

1. Dispositif d'essai rapide immunochromatographique pour la détection de plus d'une cible dans un échantillon comprenant
(a) un premier tampon de libération de conjugués constitués d'or colloïdal, comprenant au moins deux conjugués constitués d'or colloïdal I et II, dans lequel le conjugué constitué d'or colloïdal I est conjugué avec un premier anticorps capturant un antigène cible à partir d'un premier site et au moins un oligonucléotide, et dans lequel le conjugué constitué d'or colloïdal II est conjugué avec un premier antigène qui est capturé par un anticorps cible ou un deuxième anticorps qui est spécifique audit anticorps cible et au moins un oligonucléotide, dans lequel l'oligonucléotide du conjugué constitué d'or colloïdal II est différent de l'oligonucléotide du conjugué constitué d'or colloïdal I
dans lequel les conjugués constitués d'or colloïdal I et II forment un complexe "cible-conjugué d'or I" et "cible-conjugué d'or II" ;
(b) un deuxième tampon de libération de conjugués constitués d'or colloïdal, comprenant au moins deux conjugués constitués d'or colloïdal III et IV, dans lequel le conjugué constitué d'or colloïdal III est conjugué avec un troisième anticorps capturant l'antigène cible à partir d'un deuxième site et au moins un oligonucléotide, dans lequel l'oligonucléotide du conjugué constitué d'or colloïdal III est complémentaire à l'oligonucléotide du conjugué constitué d'or colloïdal I, et dans lequel le conjugué constitué d'or colloïdal IV est conjugué avec un deuxième antigène qui est capturé par le même anticorps cible qui capture le premier antigène ou un quatrième anticorps qui est spécifique audit anticorps cible et au moins un oligonucléotide, dans lequel l'oligonucléotide du conjugué constitué d'or colloïdal IV est complémentaire à l'oligonucléotide du conjugué constitué d'or colloïdal II ;
dans lequel les deux tampons de libération de conjugués constitués d'or colloïdal sont situés à des positions différentes au sein du dispositif d'essai ;
(c) une bande d'essai comprenant un tampon à échantillon, un tampon de conjugué comprenant ledit premier tampon de libération de conjugués constitués d'or colloïdal, une membrane comprenant deux zones d'essai de capture et une zone de contrôle négatif, et un tampon absorbant ;
dans lequel une zone d'essai de capture comprend ledit troisième anticorps et dans lequel l'autre zone d'essai de capture comprend ledit deuxième antigène ou quatrième anticorps ;
de sorte que après capture des "cible-conjugué d'or I" et "cible-conjugué d'or II" dans les deux zones d'essai de capture, le deuxième tampon de libération de conjugués constitués d'or va libérer les conjugués constitués d'or colloïdal III et IV.

2. Dispositif d'essai rapide immunochromatographique pour la détection de plus d'une cible dans un échantillon comprenant
(a) un premier tampon de libération de conjugués constitués d'or colloïdal, comprenant au moins deux conjugués constitués d'or colloïdal I et II, dans lequel le conjugué constitué d'or colloïdal I est conjugué avec un premier anticorps capturant un antigène cible à partir d'un premier site et au moins un autre anticorps ou antigène (A-1), et dans lequel le conjugué constitué d'or colloïdal II est conjugué avec un premier antigène qui est capturé par un anticorps cible ou un deuxième anticorps qui est spécifique audit anticorps cible et au moins un autre anticorps ou antigène (A-2), dans lequel lesdits anticorps ou antigènes (A-1) et (A-2) sont différents entre eux et sont différents des premier et deuxième antigènes et anticorps ;
dans lequel les conjugués constitués d'or colloïdal I et II forment des complexes "cible-conjugué d'or I" et "cible-conjugué d'or II" ;
(b) un deuxième tampon de libération de conjugués constitués d'or colloïdal, comprenant au moins deux conjugués constitués d'or colloïdal III et IV, dans lequel le conjugué constitué d'or colloïdal III est conjugué avec un troisième anticorps capturant l'antigène cible à partir d'un deuxième site et au moins un autre anticorps ou antigène (A-3), dans lequel ledit anticorps ou antigène (A-3) est complémentaire audit anticorps ou antigène (A-1), et dans lequel le conjugué constitué d'or colloïdal IV est conjugué avec un deuxième antigène qui est capturé par le même anticorps cible qui capture le premier antigène ou un quatrième anticorps qui est spécifique audit anticorps cible et au moins un autre anticorps ou antigène (A-4), dans lequel ledit anticorps ou antigène (A-4) est complémentaire audit anticorps ou antigène (A-2) ;
dans lequel les deux tampons de libération de conjugués constitués d'or colloïdal sont situés à des positions différentes au sein du dispositif d'essai ;
(c) une bande d'essai comprenant un tampon à échantillon, un tampon de conjugué comprenant ledit premier tampon de libération de conjugués constitués d'or colloïdal, un tampon de conjugué comprenant ledit deuxième tampon de libération de conjugués constitués d'or colloïdal, une membrane comprenant deux zones d'essai de capture et une zone de contrôle négatif, et un tampon absorbant ;
dans lequel une zone d'essai de capture comprend ledit troisième anticorps et dans lequel l'autre zone d'essai de capture comprend ledit deuxième antigène ou quatrième anticorps ;
de sorte que après capture des "cible-conjugué d'or I" et "cible-conjugué d'or II", dans les deux zones d'essai de capture, le deuxième tampon de libération de conjugués constitués d'or va libérer les conjugués constitués d'or colloïdal III et IV.

3. Dispositif selon la revendication 1 ou 2, dans lequel ladite membrane est attachée au moyen d'un adhésif à un support.

4. Dispositif selon l'une quelconque des revendications 1 à 3, dans lequel lesdits premier et deuxième tampons de conjugués constitués d'or sont stratifiés entre le tampon à échantillon et la membrane, dans lequel lesdits deux conjugués constitués d'or sont séparés par un séparateur.

5. Dispositif selon l'une quelconque des revendications 1 à 3, dans lequel ledit premier tampon de conjugué constitué d'or est attaché entre le tampon à échantillon et la membrane alors que le deuxième tampon de conjugué constitué d'or se trouve dans la partie supérieure du coffret plastique.

6. Dispositif selon l'une quelconque des revendications 3 à 5, dans lequel ledit support est un support plastique.

7. Dispositif selon l'une quelconque des revendications 1 à 6, dans lequel ladite membrane est une membrane de nitrocellulose.

8. Dispositif selon l'une quelconque des revendications 1 à 7, dans lequel lesdits premier et troisième anticorps sont sélectionnés dans le groupe composé d'un anticorps de souris anti-protéine p24 du VIH, d'un anticorps de souris anti-HBsAg, d'un anticorps anti-hlgG, d'un anticorps anti-Lipoarabinomannan, d'un anticorps dirigé contre l'antigène d'anti H. pylori, d'un anticorps dirigé contre l'antigène de Leishmania, d'un anticorps dirigé contre l'antigène de Pneumonia, d'un anticorps dirigé contre l'antigène de la Malaria, d'un anticorps dirigé contre l'antigène de Chlamydia, d'un anticorps dirigé contre l'antigène de Toxoplasma, d'un anticorps dirigé contre l'antigène de Schistosoma, d'un anticorps anti-VIH-1, et d'un anticorps anti-VIH-2.

9. Dispositif selon l'une quelconque des revendications 1 à 7, dans lequel lesdits premier et deuxième antigènes sont sélectionnée dans le groupe composé d'un conjugué d'un antigène du VIH, d'un conjugué d'un antigène de l'hépatite C, d'un antigène du VIH-1 (antigène p160 du VIH), d'un antigène du VIH-2 (antigène p36 du VIH), d'un antigène de l'Hépatite B, d'un antigène de Lipoarabinomannan, d'un antigène d'H. pylori, d'un antigène de Toxoplasma.

10. Dispositif selon l'une quelconque des revendications précédentes, dans lequel ladite zone de contrôle comprend un anticorps de capture non spécifique et/ou une protéine de capture d'anticorps non spécifique.

11. Dispositif selon la revendication 10, dans lequel ledit anticorps non spécifique est sélectionné dans le groupe composé d'un anticorps anti-IgG de souris, d'un anticorps anti-IgG de lapin, d'un anticorps anti-IgG de chèvre, d'un anticorps anti-IgG de l'âne, d'un anticorps anti-IgG de mouton, d'un anticorps anti protéine p24 du VIH, d'un anticorps anti-Lipoarabinomannan, d'un anticorps dirigé contre l'antigène d'anti H. pylori, d'un anticorps dirigé contre l'antigène de Leishmania, d'un anticorps dirigé contre l'antigène de Pneumonia, d'un anticorps dirigé contre l'antigène de la Malaria, d'un anticorps dirigé contre l'antigène de Chlamydia, d'un anticorps dirigé contre l'antigène de Toxoplasma, d'un anticorps dirigé contre l'antigène de Schistosoma, d'un anticorps anti-VIH-1, et d'un anticorps anti-VIH-2.

12. Dispositif selon la revendication 10, dans lequel ladite protéine de capture non spécifique sélectionnée est soit la Protéine A ou la Protéine G.

13. Procédé pour la fabrication d'un dispositif selon l'une quelconque des revendications 1 et 3 à 12 comprenant les étapes qui consistent à
a) préparer une solution constituée d'or colloïdal ;
b) préparer un tampon de conjugaison ;
c) répartir le tampon de conjugaison en le divisant dans un premier, un deuxième, un troisième et un quatrième flacons ;
d) ajouter un anticorps selon la revendication 8 au tampon de conjugaison dans le premier flacon ;
e) ajouter un antigène selon la revendication 9 au tampon de conjugaison dans le deuxième flacon ;
f) ajouter un anticorps selon la revendication 8 au tampon de conjugaison dans le troisième flacon, dans lequel ledit anticorps est différent de l'anticorps utilisé à l'étape d) ;
g) ajouter un antigène selon la revendication 9 au tampon de conjugaison dans le quatrième flacon, dans lequel ledit antigène est différent de l'antigène utilisé dans l'étape e) ;
h) préparer et ajouter une solution aqueuse de sérum-albumine bovin, BSA, à marquage d'oligonucléotides au premier flacon ;
i) préparer et ajouter une solution aqueuse de sérum-albumine bovin, BSA, à marquage d'oligonucléotides au deuxième flacon, dans lequel lesdits oligonucléotides sont différents des oligonucléotides utilisés à l'étape h) ;
j) préparer et ajouter une solution aqueuse de sérum-albumine bovin, BSA, à marquage d'oligonucléotides au troisième flacon, dans lequel lesdits oligonucléotides sont complémentaires aux oligonucléotides utilisés à l'étape h) ;
k) préparer et ajouter une solution aqueuse de sérum-albumine bovin, BSA, à marquage d'oligonucléotides au quatrième flacon, dans lequel lesdits oligonucléotides sont complémentaires aux oligonucléotides utilisés à l'étape i) ;
l) ajouter une solution constituée d'or colloïdal dans chaque flacon ;
m) ajouter un tampon de stabilisation dans chaque flacon ;
n) concentrer chaque conjugué ;
o) mélanger les conjugués des premier et deuxième flacons ; ajouter un tensioactif et tremper un tampon de conjugué en feuille en fibre de verre dans le conjugué ;
p) mélanger les conjugués des troisième et quatrième flacons ; tremper un autre tampon de conjugué en feuille en fibre de verre dans le conjugué ;
q) imprimer des lignes de capture/d'échantillon et de contrôle sur la membrane,
r) stratifier des cartes ; et
s) couper les cartes en bandes.

14. Procédé de fabrication d'un dispositif selon l'une quelconque des revendications 2 à 12, comprenant les étapes qui consistent à
a) préparer une solution constituée d'or colloïdal ;
b) préparer un tampon de conjugaison ;
c) répartir le tampon de conjugaison en le divisant dans un premier, un deuxième, un troisième et un quatrième flacons ;
d) ajouter un anticorps selon la revendication 8 au tampon de conjugaison dans le premier flacon ;
e) ajouter un antigène selon la revendication 9 au tampon de conjugaison dans le deuxième flacon ;
f) ajouter un anticorps selon la revendication 8 au tampon de conjugaison dans le troisième flacon, dans lequel ledit anticorps est différent de l'anticorps utilisé à l'étape d) ;
g) ajouter un antigène selon la revendication 9 au tampon de conjugaison dans le quatrième flacon, dans lequel ledit antigène est différent de l'antigène utilisé à l'étape e) ;
h) préparer et ajouter une solution aqueuse comprenant des anticorps ou antigènes au premier flacon, dans lequel lesdits anticorps ou antigènes sont différents des anticorps ou antigènes utilisés aux étapes d), e), g) et f) ;
i) préparer et ajouter une solution aqueuse comprenant des anticorps ou antigènes au deuxième flacon, dans lequel lesdits anticorps ou antigènes sont différents des anticorps ou antigènes utilisés aux étapes d), e), g) et f) et des anticorps utilisés à l'étape h) ;
j) préparer et ajouter une solution aqueuse comprenant des anticorps ou antigènes au troisième flacon, dans lequel lesdits anticorps ou antigènes sont complémentaires aux anticorps ou antigènes utilisés à l'étape h) ;
k) préparer et ajouter une solution aqueuse comprenant des anticorps ou antigènes au quatrième flacon, dans lequel lesdits anticorps ou antigènes sont complémentaires aux anticorps ou antigènes utilisés à l'étape i) ;
l) ajouter une solution constituée d'or colloïdal dans chaque flacon ;
m) ajouter un tampon de stabilisation dans chaque flacon ;
n) concentrer chaque conjugué ;
o) mélanger les conjugués des premier et deuxième flacons ; ajouter un tensioactif et tremper un tampon de conjugué en feuille en fibre de verre dans le conjugué ;
p) mélanger les conjugués des troisième et quatrième flacons ; tremper un autre tampon de conjugué en feuille en fibre de verre dans le conjugué ;
q) imprimer des lignes de capture/d'échantillon et de contrôle sur la membrane,
r) stratifier des cartes ; et
s) couper les cartes en bandes.

15. Utilisation d'un dispositif selon l'une quelconque des revendications 1 à 12 pour la détection d'au moins deux maladies dans au moins un échantillon.

16. Utilisation selon la revendication 15, dans laquelle deux maladies sont détectées en même temps dans un échantillon.

17. utilisation selon la revendication 15 ou 16, dans laquelle ledit échantillon a été obtenu d'un être humain.

18. Utilisation selon la revendication 15, dans laquelle ledit échantillon est sélectionné dans le groupe composé de sang total, de sérum, de plasma, de salive et d'urine.

19. Utilisation selon l'une quelconque des revendications 15 à 18, dans laquelle ladite maladie détectée dans ledit échantillon est sélectionnée dans le groupe composé du VIH, de l'hépatite A, de l'hépatite B, de l'hépatite C, d'une infection par H. pylori, de la Leishmania, de la Schistosomiasis, de la malaria, de la pneumonie, de la toxoplasmose, de la tuberculose et de l'infection de Chlamydia.

20. Trousse pour la détection d'une maladie comprenant le dispositif selon l'une quelconque des revendications 1 à 12 et un manuel.

21. Trousse selon la revendication 20 comprenant en outre un tampon de dosage.

22. Trousse selon la revendication 21, dans laquelle ledit tampon de dosage comprend un agent de conservation.
